# EUROPEAN PATENT SPECIFICATION

(11) **EP 4 650 628 B1**
(45) Date of publication and mention of the grant of the patent: **28.10.2026**
(21) Application number: 24853037.0
(22) Date of filing: 07.08.2024
(51) Int. Cl.: F16J 15/16, A61M 60/13, A61M 60/237, A61M 60/414, A61M 60/829, A61B 17/32, A61B 17/3207, A61B 17/00

(54) **TRANSMISSION SEALING STRUCTURE**
GETRIEBEDICHTUNGSSTRUKTUR
STRUCTURE D'ÉTANCHÉITÉ DE TRANSMISSION

(30) Priority: 19.03.2024 CN 202410309235
(43) Date of publication of application: 19.11.2025
(73) Proprietor: Shanghai EnginPrime Medtec CO., LTD., Shanghai 200032 (CN)
(72) Inventor: ZHENG, Xiaojuan, Chengdu, Sichuan 610095 (CN); ZHENG, Tao, Chengdu, Sichuan 610095 (CN)
(74) Representative: M. Zardi & Co S.A.
(86) International application number: PCT/CN2024/110244
(87) International publication number: WO 2025/194662

(56) References cited:
- WO-A1-2023/134695
- WO-A1-2023/283751
- CN-A- 101 149 060
- CN-A- 104 251 215
- CN-U- 219 323 783
- CN-U- 219 662 648
- US-A1- 2016 074 057

## Description

### TECHNICAL FIELD

The present disclosure relates to the technical field of transmission sealing, and in particular to a transmission sealing structure.

### BACKGROUND

For interventional instruments in which the mechanical transmission source is located outside the body and functional elements need to be located inside the body, such as OCT (Optical Coherence Tomography), a thrombus clearing device through rotary grinding, a thrombectomy device and a catheter pump, the functional elements communicate with blood vessels to be in contact with blood. Moreover, the functional elements need to rotate at a high speed inside the body. The blood may permeate into the mechanical transmission source outside the body through structural gaps due to the effect of blood pressure. In the prior art, the rotation of the functional elements is realized through a bearing or a shaft sleeve, and sealing is realized through a sealing structure, so that the blood is avoided from entering the mechanical transmission source. The device is complex in structure and high in manufacturing cost.

In addition, the transmission sealing structure refers to gas sealing, fluid sealing or mechanical sealing (rubber rings, etc.), and there are many problems in the prior art. First, the structure is easy to fail. Second, the requirements for sealing conditions, such as temperature and pressure, are relatively strict. Third, the service life is short.

WO 2023/283751 A1 discloses a device for assisting the heart in the event of heart failure, the device comprising: a catheter; a drive shaft arranged in the catheter in a penetrating manner; and a pump which can be delivered to a desired location in the heart via the catheter to pump blood. The pump comprises: a pump housing connected to a distal end of the catheter and having an inlet end and an outlet end, and an impeller accommodated in the pump housing, wherein the impeller is driven by the drive shaft to draw blood into the pump housing from the inlet end and discharge same from the outlet end; and the pump housing and the impeller are configured such that the pump is in a compressed state when in an interventional configuration to enable the pump, which is within a first outer diameter, to be delivered to the vascular system of a subject, and the pump is in a deployed state when in an operating configuration to enable the pump, which is within a second outer diameter that is greater than the first outer diameter, to pump blood to a desired location.

CN 219 662 648 U discloses a blood pumping catheter, the blood pumping catheter comprises a driving motor. A transmission system comprises a coaxial and nested active transmission component and a passive transmission component, with a gap between the active transmission component and the passive transmission component. The active transmission component is fixedly connected to the output shaft of the driving motor, and the active transmission component comprises at least two active magnets of opposite polarity. The passive transmission component comprises at least two passive magnets of opposite polarity.

### SUMMARY

The invention is defined in the appended claims.

Some embodiments aim to provide a transmission sealing structure so as to solve the problems in the prior art. The transmission sealing structure is simple in structure, high in reliability, long in service life and low in cost. A sealing cover is matched with an internal magnetic component and an external magnetic component to realize detachable magnetic torque transmission, so that a mechanical transmission source and an interventional transmission functional element are detachable, the mechanical transmission source may be reused, and the cost of interventional instruments is reduced.

In order to achieve the above-mentioned purpose, some embodiments provide the following solutions.

The present disclosure provides a transmission sealing structure, including an external magnetic component, an internal magnetic component, a driven shaft and a sealing cover, where the internal magnetic component is provided on the driven shaft, the external magnetic component is provided at an outer side of the internal magnetic component, the sealing cover has an opening end that is hermetically connected with a perfusion chamber by adhesion and an outer wall that is hermetically connected with the perfusion chamber through interference fit so as to isolate the internal magnetic component from the external magnetic component, and the external magnetic component is configured to rotate to drive the internal magnetic component and the driven shaft to rotate with respect to the sealing cover. The internal magnetic component is sleeved on the driven shaft and is fixedly connected with the driven shaft, the sealing cover is provided at the outer side of the internal magnetic component, the external magnetic component is provided at an outer side of the sealing cover, and both the driven shaft and the internal magnetic component are movable in an axial direction of the sealing cover. One end of the driven shaft protrudes from the opening end of the sealing cover, there is a gap formed between the driven shaft and the perfusion chamber, there is a gap formed between the driven shaft and the sealing cover, and between the internal magnetic component and the sealing cover. Fluid flows to a space formed by the driven shaft, the internal magnetic component and the sealing cover from the gap between the driven shaft and the perfusion chamber, and then flows out from the gap between the driven shaft and the perfusion chamber.

Preferably, one end of the driven shaft protrudes from the opening end of the sealing cover, there is a gap formed between the driven shaft and the perfusion chamber, an other end of the driven shaft is adjacent to a sealing end of the sealing cover, and there is a gap formed between the driven shaft and the sealing cover, and there is a gap formed between the internal magnetic component and the sealing cover.

Preferably, the driven shaft is a solid shaft, and one end of the driven shaft is connected with a transmission shaft.

Preferably, the driven shaft is a hollow shaft, a transmission shaft extends into the driven shaft, and the transmission shaft is a hollow shaft or a solid shaft.

Preferably, the driven shaft is a plain shaft.

Preferably, the driven shaft includes a first shaft part and a second shaft part, the first shaft part is located inside the sealing cover, the second shaft part is located outside the sealing cover, a spiral structure is provided at an outer side of the second shaft part, and a rotation direction of the spiral structure is opposite to a spiral direction of the driven shaft.

Preferably, each of the internal magnetic component and the external magnetic component includes magnetic rings, the magnetic rings in the internal magnetic component are provided in an axial direction of the driven shaft or the magnetic rings in the internal magnetic component are sequentially nested in a radial direction of the driven shaft, the magnetic rings in the external magnetic component are provided in the axial direction of the driven shaft or the magnetic rings in the external magnetic component are sequentially nested in the radial direction of the driven shaft.

Preferably, both the internal magnetic component and the external magnetic component include several magnetic strips uniformly provided in a circumferential direction of the driven shaft.

Preferably, a wear-resistant insert is provided between the driven shaft and the perfusion chamber, the wear-resistant insert is fixedly connected with the perfusion chamber, the wear-resistant insert is rotatable with respect to the driven shaft, and the wear-resistant insert is capable of axially limiting the driven shaft.

Compared with the prior art, some embodiments have the following technical effects.

The sealing cover in the present disclosure may realize a sealing effect, and also may play a role of a shaft sleeve to realize integrated design of sealing and rotary connection, so that the structure is simpler. In the transmission process, the external magnetic component is not in contact with the internal magnetic component. Moreover, the driven shaft may realize rotation, and also may move in the axial direction of the driven shaft. The transmission sealing structure in some embodiments is simple in structure, high in reliability, long in service life and low in cost. The sealing cover is matched with the internal magnetic component and the external magnetic component to realize detachable magnetic torque transmission, so that the mechanical transmission source and the interventional transmission functional element are detachable, the mechanical transmission source may be reused, and the cost of interventional instruments is reduced.

### BRIEF DESCRIPTION OF THE DRAWINGS

In order to explain the embodiments of the present disclosure or the technical scheme in the prior art more clearly, the drawings needed in the embodiments will be briefly introduced hereinafter. Obviously, the drawings in the following description are only some embodiments of the present disclosure. Other drawings can be obtained according to these drawings without paying creative labor for those skilled in the art.
FIG. 1 is a schematic diagram of a catheter pump.
FIG. 2 is a schematic diagram of a distal end of a catheter pump (having no sheath cover and no switching block).
FIG. 3 is a schematic diagram of the connection between a housing and a multi-cavity pipe.
FIG. 4 is a schematic diagram of a multi-cavity pipe.
FIG. 5 is a first schematic diagram of an application of a transmission sealing structure according to the present disclosure.
FIG. 6 is a first schematic diagram of the connection between a pigtail pipe and an impeller rotating shaft.
FIG. 7 is a second schematic diagram of the connection between a pigtail pipe and an impeller rotating shaft.
FIG. 8 is a schematic diagram of an application of an impeller structure.
FIG. 9 is a first schematic diagram of an impeller rotating shaft.
FIG. 10 is a partial enlarged view of position I in FIG. 9.
FIG. 11 is a second schematic diagram of an impeller rotating shaft.
FIG. 12 is a partial enlarged view of position II in FIG. 11.
FIG. 13 is a third schematic diagram of an impeller rotating shaft.
FIG. 14 is a partial enlarged view at position III in FIG. 13.
FIG. 15 is a fourth schematic diagram of an impeller rotating shaft.
FIG. 16 is a partial enlarged view at position IV in FIG. 15.
FIG. 17 is a schematic diagram of a hub passing through a through hole.
FIG. 18 is a cross-sectional view of an impeller rotating shaft.
FIG. 19 is a cross-sectional view of an application of an impeller structure.
FIG. 20 is a front view of an impeller rotating shaft.
FIG. 21 is a schematic diagram of the bending of an impeller rotating shaft.
FIG. 22 is a schematic diagram of the connection between a proximal end of an impeller rotating shaft and a transmission shaft.
FIG. 23 is a first schematic diagram of a multi-cavity pipe.
FIG. 24 is a schematic diagram of perfusion liquid flowing in a perfusion cavity, a third channel, a liquid path channel and an outflow cavity.
FIG. 25 illustrates perfusion liquid entering a human body through a liquid path channel (an impeller rotating shaft having no rotating shaft step).
FIG. 26 is a schematic diagram of a distal end of a catheter pump (having a sheath cover and a switching block).
FIG. 27 is a schematic diagram of the connection between a proximal end of an impeller rotating shaft and a switching block.
FIG. 28 is a side view of the connection between a proximal end of an impeller rotating shaft and a switching block.
FIG. 29 is a first schematic diagram of sliding between a proximal end of an impeller rotating shaft and a switching block.
FIG. 30 is a second schematic diagram of sliding between a proximal end of an impeller rotating shaft and a switching block.
FIG. 31 is a schematic diagram of the connection between a proximal end of an impeller rotating shaft and a switching block.
FIG. 32 is an external schematic diagram of an impeller rotating shaft, a sheath cover and a transmission shaft.
FIG. 33 is an internal schematic diagram of an impeller rotating shaft, a sheath cover and a transmission shaft.
FIG. 34 is a partial enlarged view of an impeller rotating shaft, a sheath cover and a transmission shaft.
FIG. 35 is a schematic diagram of the contraction of an expandable bracket.
FIG. 36 is a schematic diagram of the expansion of an expandable bracket.
FIG. 37 is a schematic diagram of perfusion liquid flowing in a perfusion cavity, a first channel, a third channel, a liquid path channel and an outflow cavity.
FIG. 38 is a schematic diagram of perfusion liquid flowing in a perfusion cavity, a first channel and a liquid path channel.
FIG. 39 is a schematic diagram of perfusion fluid flowing in a perfusion cavity and a third channel.
FIG. 40 is a schematic diagram of perfusion fluid flowing in a perfusion cavity and an outflow cavity.
FIG. 41 is a first schematic diagram of a sealing cover according to the present disclosure.
FIG. 42 is a second schematic diagram of a sealing cover according to the present disclosure.
FIG. 43 is a first schematic diagram of the connection between a sealing cover and a perfusion chamber according to the present disclosure.
FIG. 44 is a second schematic diagram of the connection between a sealing cover and a perfusion chamber according to the present disclosure.
FIG. 45 is a schematic diagram of the connection between an internal magnetic component and a driven shaft according to the present disclosure.
FIG. 46 is a first schematic diagram of a driven shaft moving in axial direction of a driven shaft according to the present disclosure.
FIG. 47 is a second schematic diagram of a driven shaft moving in axial direction of a driven shaft according to the present disclosure.
FIG. 48 is a schematic diagram of a driven shaft, an internal magnetic component and a sealing cover according to the present disclosure before installation.
FIG. 49 is a schematic diagram of a driven shaft, an internal magnetic component and a sealing cover according to the present disclosure after installation.
FIG. 50 is a schematic diagram of an external magnetic component and an external magnetic component mounting sleeve according to the present disclosure before installation.
FIG. 51 is a schematic diagram of an external magnetic component driving an internal magnetic component to rotate according to the present disclosure.
FIG. 52 is a schematic diagram of an external magnetic component driving an internal magnetic component to rotate according to the present disclosure.
FIG. 53 is a schematic diagram of a flow direction of perfusion liquid in a transmission sealing structure in FIG. 5 according to the present disclosure.
FIG. 54 is a second schematic diagram of an application of a transmission sealing structure.
FIG. 55 is a schematic diagram of a flow direction of perfusion liquid in a transmission sealing structure in FIG. 54.
FIG. 56 is a third schematic diagram of an application of a transmission sealing structure according to the present disclosure.
FIG. 57 is a partial enlarged view of a spiral structure according to the present disclosure.
FIG. 58 is a schematic diagram of a driven shaft (having a spiral structure) according to the present disclosure.
FIG. 59 is a schematic diagram of a flow direction of perfusion liquid in a transmission sealing structure in FIG. 56 according to the present disclosure.
FIG. 60 is a fourth schematic diagram of an application of a transmission sealing structure according to the present disclosure.
FIG. 61 is a schematic diagram of a flow direction of perfusion liquid in a transmission sealing structure in FIG. 60 according to the present disclosure.
FIG. 62 is a fifth schematic diagram of an application of a transmission sealing structure according to the present disclosure.
FIG. 63 is a schematic diagram of a flow direction of perfusion liquid in a transmission sealing structure in FIG. 62 according to the present disclosure.
FIG. 64 is a second schematic diagram of a multi-cavity pipe.
FIG. 65 is a side view of a multi-cavity pipe.
FIG. 66 is a sixth schematic diagram of an application of a transmission sealing structure according to the present disclosure.
FIG. 67 is a schematic diagram of a flow direction of perfusion liquid in a transmission sealing structure in FIG. 66 according to the present disclosure.
FIG. 68 is a seventh schematic diagram of a proximal end of a catheter pump.
FIG. 69 is a schematic diagram of a flow direction of perfusion fluid in a transmission sealing structure in FIG. 68.
FIG. 70 illustrates perfusion liquid entering a human body through a liquid path channel (an impeller rotating shaft having a rotating shaft step).
FIG. 71 is a schematic diagram of a fit gap between a hydraulic cavity and a rotating shaft.

In the figures: 1 pigtail pipe; 2 impeller structure; 3 expandable bracket; 4 housing; 5 multi cavity pipe; 6 handle module; 7 impeller rotating shaft; 8 impeller; 9 hub; 10 blade; 11 liquid path channel; 12 transmission shaft; 13 blood inlet window; 14 blood outlet window; 15 rotating structure; 16 rotating shaft step; 17 groove; 18 through hole; 19 film layer; 20 first rigid section; 21 flexible section; 22 second rigid section; 23 perfusion cavity; 24 outflow cavity; 25 inlet; 26 inner pipe; 27 outer pipe; 28 spring pipe; 29 switching block; 30 first channel; 31 protrusion; 32 chute; 33 second channel; 34 sheath cover; 35 opening; 36 sheath cover limiting step; 37 third channel; 38 perfusion chamber; 39 pressure measuring hole; 40 driving motor; 41 external magnetic component; 42 internal magnetic component; 43 driven shaft; 44 sealing cover; 45 proximal end insert; 46 external magnetic component mounting sleeve; 47 opening end; 48 sealing end; 50 first shaft part; 51 second shaft part; 52 spiral structure; 53 wear-resistant insert; 54 projection; 55 first diffusion stress pipe; 56 motor housing; 57 second diffusion stress pipe; 58 cable; 59 perfusion pipe; 60 outflow pipe; 61 locking nut; 62 pressure measuring cavity; 63 partition plate; 64 detection cavity; 65 pressure measuring pipe; 66 pressure measuring element; 67 inner ring of a bearing; 68 outer ring of a bearing; 70 first connecting part; 71 second connecting part; 72 plug; 73 shaft sleeve; 74 transmission line; 75 transmission line antenna; 76 hydraulic cavity; 77 rotating shaft fit gap.

### DETAILED DESCRIPTION OF THE EMBODIMENTS

The technical scheme in the embodiment of the present disclosure will be described clearly and completely with reference to the drawings hereinafter. Obviously, the described embodiments are only some embodiments of the present disclosure, rather than all of the embodiments. Based on the embodiments in the present disclosure, all other embodiments obtained by those skilled in the art without paying creative labor belong to the scope of protection of the present disclosure.

The present disclosure aims to provide a transmission sealing structure so as to solve the problems in the prior art. The transmission sealing structure is simple in structure, high in reliability, long in service life and low in cost. A sealing cover is matched with an internal magnetic component and an external magnetic component to realize detachable magnetic torque transmission, so that a mechanical transmission source and an interventional transmission functional element are detachable, the mechanical transmission source may be reused, and the cost of interventional instruments is reduced.

In order to make the above purpose, features and advantages of the present disclosure more obvious and understandable, the present disclosure will be further described in detail with reference to the drawings and the detailed description.

### Embodiment 1

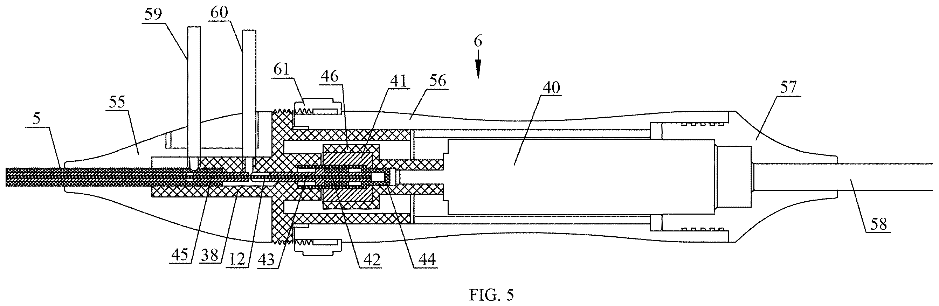

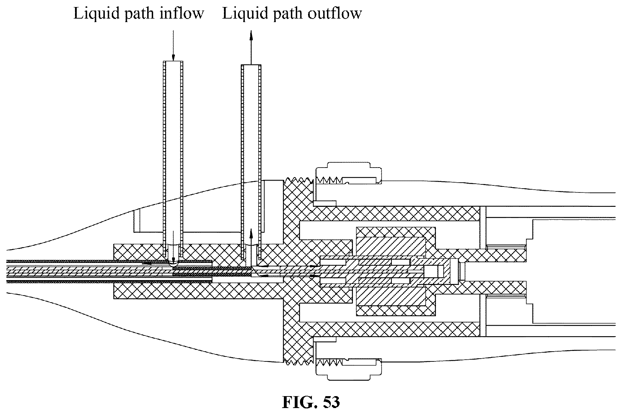

As shown in FIG. 5, FIG. 41 and FIG. 53, the embodiment provides a transmission sealing structure. The transmission sealing structure includes an external magnetic component 41, an internal magnetic component 42, a driven shaft 43 and a sealing cover 44. The external magnetic component 41, the internal magnetic component 42, the driven shaft 43 and the sealing cover 44 are all coaxially provided. Both the internal magnetic component 42 and the external magnetic component 41 are made of neodymium iron boron and other materials. Each of the internal magnetic component 42 and the external magnetic component 41 includes a magnetic ring. The internal magnetic component 42 is sleeved on the driven shaft 43 and is adhered to the driven shaft 43. The internal magnetic component 42 is configured to receive a magnetic torque. The sealing cover 44 is provided at an outer side of the internal magnetic component 42. The external magnetic component 41 is provided in an external magnetic component mounting sleeve 46. The external magnetic component mounting sleeve 46 is located at the inner side of the perfusion chamber 38. The external magnetic component mounting sleeve 46 is fixedly connected with the external magnetic ring and the output shaft of the driving motor 40, respectively. The external magnetic component mounting sleeve 46 is configured to transmit the rotating torque of the output shaft of the driving motor 40 and fix the external magnetic ring. The external magnetic component mounting sleeve 46 is made of engineering plastics such as Peek, PC, ABC, etc. The external magnetic component 41 is located at an outer side of the sealing cover 44. There is a gap between the external magnetic component 41 and the sealing cover 44. The sealing cover 44 is made of non-metallic materials, such as ceramics (such as alumina and zirconia), glass, diamond and other materials. Alternatively, the sealing cover 44 is made of plastic, and a non-metallic wear-resistant coating is provided outside the plastic, so that the opening end 47 of the sealing cover 44 is hermetically connected with the perfusion chamber 38 by adhesion. The outer wall of the sealing cover 44 is hermetically connected with the perfusion chamber 38 through interference fit. The sealing cover 44 is configured to isolate the internal magnetic component 42 from the external magnetic component 41. The external magnetic component 41 rotates, which in turn drives both the internal magnetic component 42 and the driven shaft 43 to rotate with respect to the sealing cover 44. The driven shaft 43 and the internal magnetic component 42 can also move in the axial direction of the sealing cover 44, so that when an interventional instrument (such as a catheter pump) is bent while entering circuitous vessels, the transmission shaft and the multi-cavity pipe may cause relative displacement, since a multi-cavity pipe and a transmission shaft are different in bending curvature. Therefore, the driven shaft 43 connected with one end of the transmission shaft needs to be in a free movement state relative to the multi-cavity pipe .

Specifically, in the embodiment, the sealing cover 44 is integrally molded. The sealing cover 44 includes a first connecting part 70 and a second connecting part 71. One end of the first connecting part 70 is an opening end 47, and the other end of the first connecting part 70 is connected with one end of the second connecting part 71. The other end of the second connecting part 71 is a closed end. The driven shaft 43 is provided with a projection 54. The projection 54 is rotatably connected with the first connecting part 70 of the sealing cover 44. The second connecting part 71 of the sealing cover 44 is rotatably connected with the driven shaft 43.

In the embodiment, the driven shaft 43 is a solid shaft, and one end of the driven shaft 43 is connected with the transmission shaft 12. Alternatively, the driven shaft 43 is a hollow shaft, the transmission shaft 12 extends into the driven shaft 43, and the transmission shaft 12 is a solid shaft.

In the embodiment, one end of the driven shaft 43 protrudes from the opening end 47 of the sealing cover 44, and there is a gap between the driven shaft 43 and the perfusion chamber 38. The other end of the driven shaft 43 is adjacent to a sealing end 48 of the sealing cover 44. The driven shaft 43 is a plain shaft. There is a gap formed between the driven shaft 43 and the perfusion chamber 38, there is a gap formed between the driven shaft 43 and the sealing cover 44, and between the internal magnetic component 42 and the sealing cover 44.

**When** the transmission sealing structure of this embodiment is used, the driving motor 40 drives the external magnetic component mounting sleeve 46 to drive the external magnetic component 41 to rotate. The external magnetic component 41 drives the internal magnetic component 42 and the driven shaft 43 to rotate through the magnetic cooperation between the external magnetic component 41 and the internal magnetic component 42. The driven shaft 43 drives the transmission shaft 12 to rotate.

Fluid flows to the space formed by the driven shaft 43, the internal magnetic component 42 and the sealing cover 44 from the gap between the driven shaft 43 and the perfusion chamber 38, and then flows out from the gap between the driven shaft 43 and the perfusion chamber 38.

The sealing cover 44 of this embodiment not only can achieve the sealing function, but also achieve the rotatable connection, so as to achieve the integrated design of sealing and rotatable connection. In the transmission process, the external magnetic component 41 is not in contact with the internal magnetic component 42. Moreover, the driven shaft 43 not only can rotate, but also move in the axial direction of the driven shaft 43. The transmission of this embodiment is simple in sealing structure, high in reliability, long in service life and low in cost.

### Embodiment 2

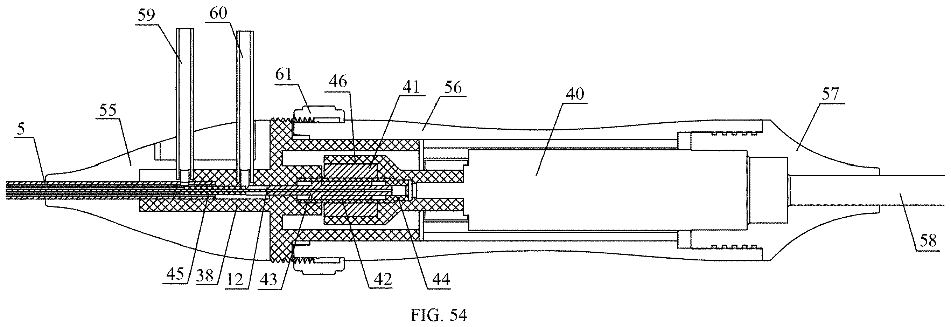

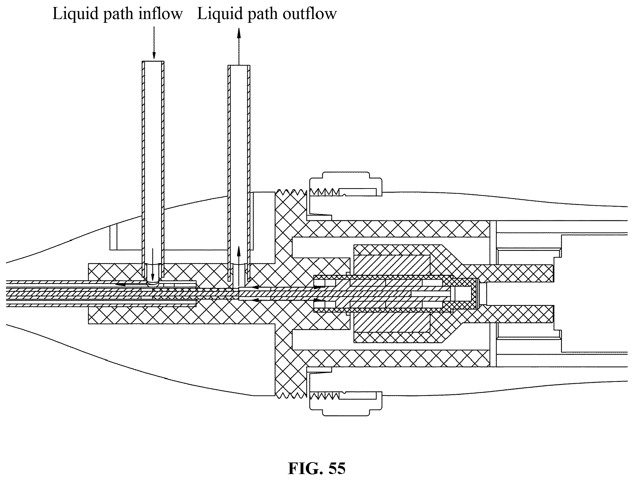

As shown in FIG. 54 and FIG. 55, the difference between this embodiment and Embodiment 1 is that in this embodiment, both the internal magnetic component 42 and the external magnetic component 41 include at least two magnetic rings. The magnetic rings in the internal magnetic component 42 are provided in the axial direction of the driven shaft 43. The magnetic rings in the external magnetic component 41 are provided in the axial direction of the driven shaft 43. By increasing the volume of the internal magnetic component 42 and the external magnetic component 41 (for example, increasing the length, the thickness, the surface area, etc.), the strength of the internal magnetic component 42 and the external magnetic component 41 is increased, thereby increasing the magnetic torque.

### Embodiment 3

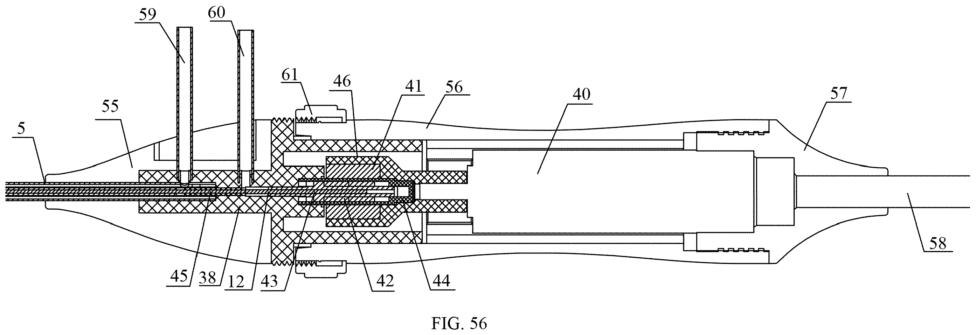

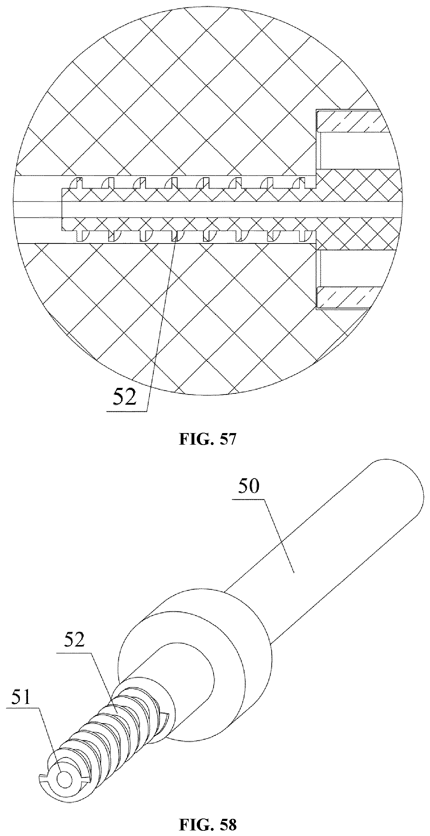

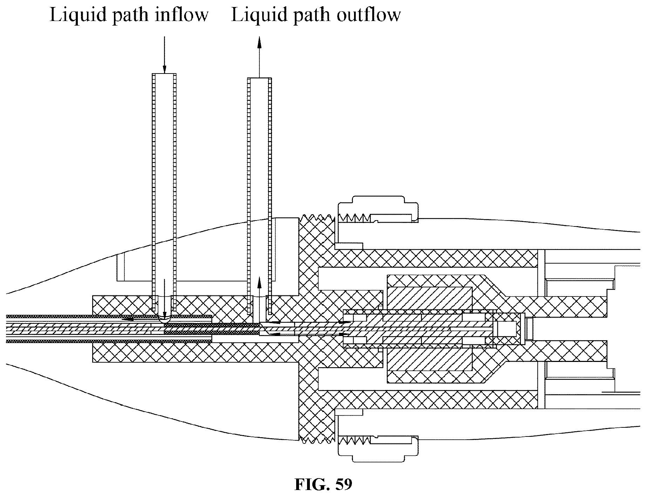

As shown in FIG. 56 to FIG. 59, the difference between this embodiment and Embodiment 2 is that in this embodiment, the driven shaft 43 includes a first shaft part 50 and a second shaft part 51. The first shaft part 50 is located inside the sealing cover 44. The second shaft part 51 is located outside the sealing cover 44. A spiral structure 52 is provided at an outer side of the second shaft part 51. A spiral direction of the spiral structure 52 is opposite to a rotation direction of the driven shaft 43. For example, if the driven shaft 43 rotates to the right, the spiral direction of spiral structure 52 is left-handed. The spiral structure 52 is provided, so that the liquid in the space formed by the driven shaft 43, the internal magnetic component 42 and the sealing cover 44 can be promoted to flow out, the friction heat and the wear particles can be taken away, blood is prevented from existing in the space formed by the driven shaft 43, the internal magnetic component 42 and the sealing cover 44, and the protein in the blood is prevented from solidifying to hinder the rotation of the driven shaft 43 and the internal magnetic component 42.

### Embodiment 4

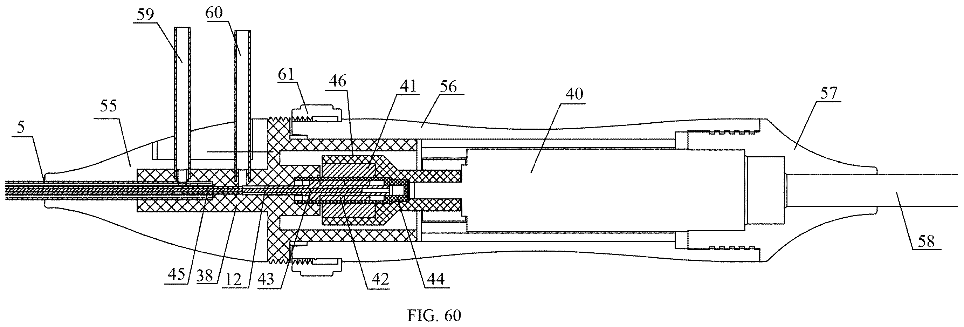

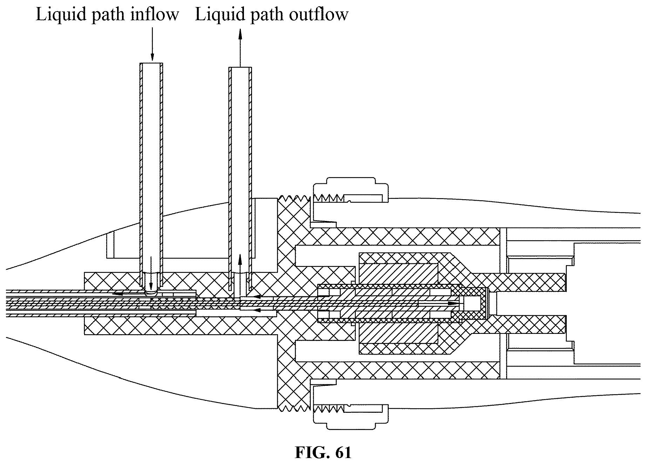

As shown in FIG. 60 and FIG. 61, the difference between this embodiment and Embodiment 3 is that in this embodiment, the transmission shaft 12 is a hollow shaft. That is, a return channel is provided inside the transmission shaft 12. Liquid is divided into two parts. One part flows to the space formed by the driven shaft 43, the internal magnetic component 42 and the sealing cover 44 from the gap between the driven shaft 43 and the perfusion chamber 38, flows out from the gap between the driven shaft 43 and the perfusion chamber 38, takes away the friction heat between the driven shaft 43 and the sealing cover 44 and then flows out through the outflow pipe. The other part flows to the space formed by the driven shaft 43, the internal magnetic component 42 and the sealing cover 44 through the return channel of the transmission shaft 12, and then flows out from the gap between the driven shaft 43 and the perfusion chamber 38.

### Embodiment 5

The difference between this embodiment and Embodiment 1 is that in this embodiment, both the internal magnetic component 42 and the external magnetic component 41 include at least two magnetic rings. All of the magnetic rings in the internal magnetic component 42 are sequentially nested in the radial direction of the driven shaft 43. All of the magnetic rings in the external magnetic component 41 are sequentially nested in the radial direction of the driven shaft 43.

### Embodiment 6

The difference between this embodiment and Embodiment 1 is that in this embodiment, both the internal magnetic component 42 and the external magnetic component 41 include several magnetic strips uniformly provided in the circumferential direction of the driven shaft 43. The number of magnetic strips of the internal magnetic component 42 is the same as or different from that of the external magnetic component 41.

### Embodiment 7

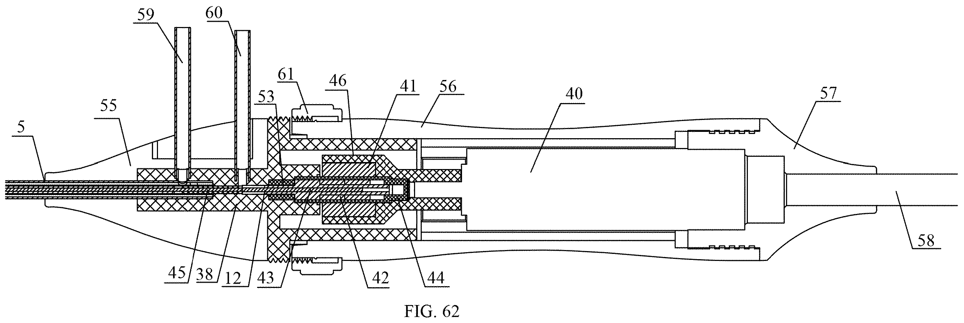

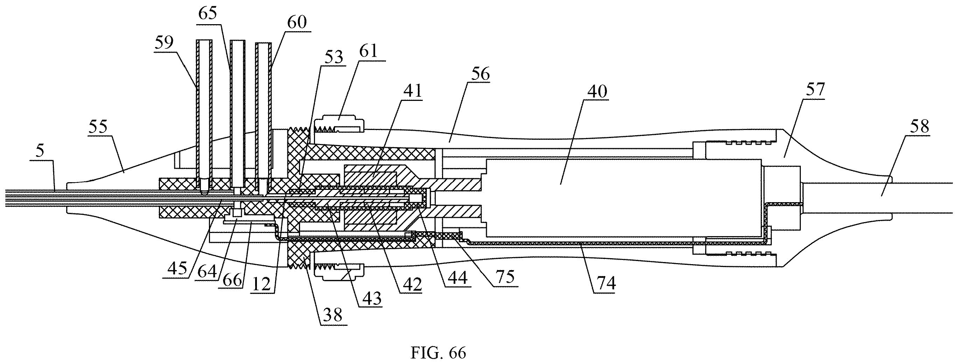

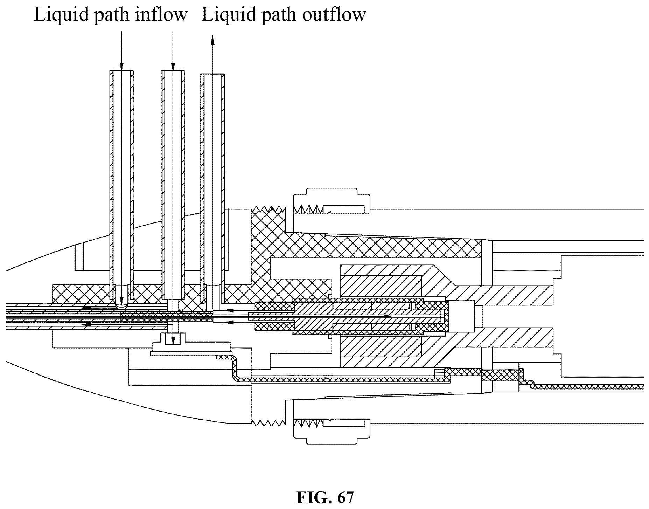

As shown in FIG. 62 to FIG. 63, and FIG. 66 to FIG. 67, the difference between this embodiment and Embodiment 1 is that in this embodiment, a wear-resistant insert 53 is provided between the driven shaft 43 and the perfusion chamber 38. The wear-resistant insert 53 is made of ceramic materials, such as alumina and zirconia. The wear-resistant insert 53 is fixedly connected with the perfusion chamber 38. When the driven shaft 43 rotates, The wear-resistant insert 53 forms bearing fit with the driven shaft 43 and is rotatable with respect to the driven shaft, and the wear-resistant insert 53 is matched with the projection 54 to axially limit the driven shaft 43.

The transmission sealing structure in Embodiment 1 to Embodiment 7 is applicable to interventional instruments in which the mechanical transmission source (the driving motor 40) is provided outside the body and functional elements need to be provided inside the body, such as an OCT, a thrombus clearing device through rotary grinding, a thrombectomy device or a catheter pump, and the functional elements need to rotate. Moreover, the sealing structure needs to avoid blood from entering into the mechanical transmission source (the driving motor 40). By using the transmission sealing structure in Embodiment 1 to Embodiment 7, the sealing cover 44 may achieve the effects of a bearing and a shaft sleeve to realize the rotation of the functional elements, and also may achieve a sealing effect, so that blood is avoided from entering into the mechanical transmission source (the driving motor 40). The transmission sealing structure is simple in structure, low in cost and high in reliability. Moreover, by using the transmission sealing structure in Embodiment 1 to Embodiment 7, the sealing cover 44 is matched with the internal magnetic component 42 and the external magnetic component 41 to realize detachable magnetic torque transmission, so that the mechanical transmission source (the driving motor 40) may be reused, and the cost of the interventional instrument is reduced.

Specific application of Embodiment 1 to Embodiment 7 is as follows.

### Application Example 1

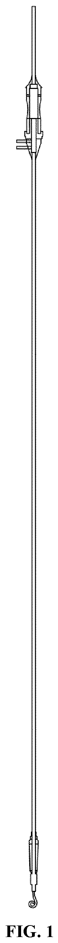

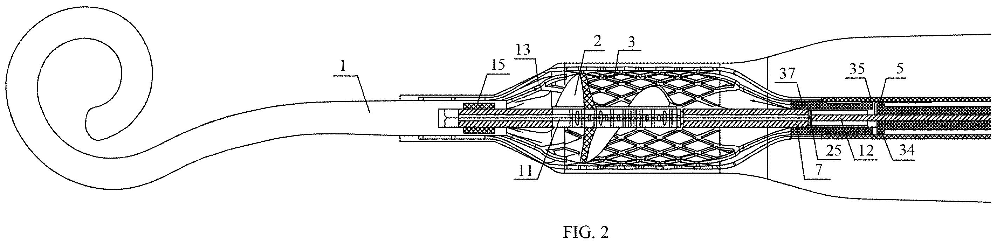

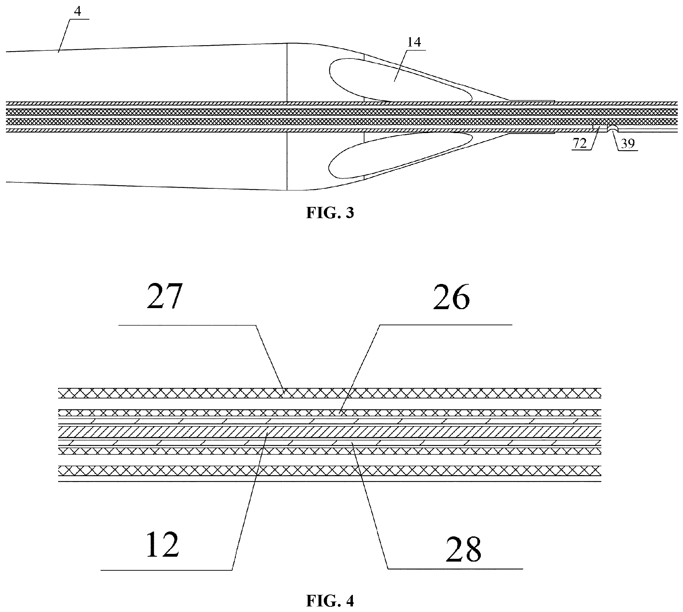

As shown in FIG. 1 to FIG. 5, this application example provides a catheter pump, which includes a pigtail pipe 1, a functional module, an expandable bracket 3, a housing 4, a liquid path module and a handle module 6. The functional module includes an impeller structure 2. The impeller structure 2 includes an impeller rotating shaft 7 and an impeller 8. The impeller rotating shaft 7 is configured to transmit a torque and drive the impeller 8 to rotate. The impeller 8 includes a hub 9 and several blades 10. The several blades 10 are provided on the hub 9. A liquid path channel 11 is provided in the impeller rotating shaft 7. The liquid path channel 11 penetrates from a proximal end of the impeller rotating shaft 7 to a distal end of the impeller rotating shaft 7. A side wall of the impeller rotating shaft 7 is provided with a connecting part. The hub 9 is connected with the connecting part by integral injection molding. The connecting part is configured to limit relative movement of the impeller 8. The impeller rotating shaft 7 is rotatably connected with the pigtail pipe 1. The liquid path module includes a multi-cavity pipe 5. The impeller rotating shaft 7 is connected with a driving motor 40 in the handle module 6 through a transmission shaft 12 in the multi-cavity pipe 5. The expandable bracket 3 is located at an outer side of the impeller structure 2. A distal end of the expandable bracket 3 is connected with the pigtail pipe 1. A proximal end of the expandable bracket 3 is connected with the multi-cavity pipe 5. The housing 4 is located at an outer side of the expandable bracket 3. The housing 4 is connected with the pigtail pipe 1 and the multi-cavity pipe 5, respectively, and the housing 4 is provided with a blood inlet window 13 and a blood outlet window 14. The blood inlet window 13 is provided adjacent to the distal end of the catheter pump, and the blood outlet window 14 is provided adjacent to the proximal end of the catheter pump.

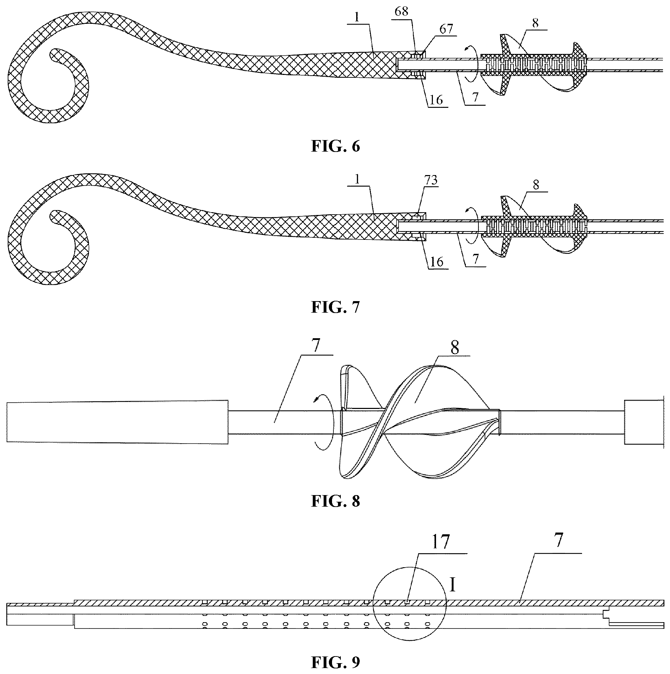

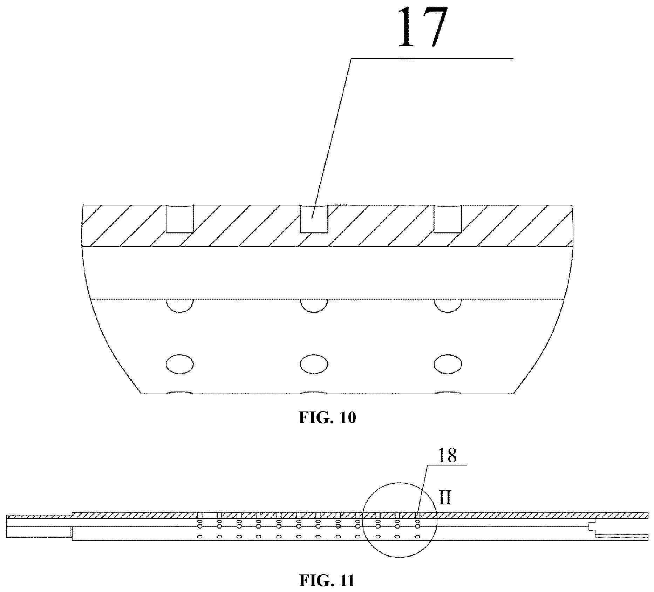

Specifically, as shown in FIG. 9 and FIG. 10, in this application example, the connecting part is a groove 17. The position of the hub 9 corresponding to the groove 17 is located in the groove 17 and attached to the surface of the groove 17, so that the movement of the impeller 8 can be restricted. Compared with the impeller rotating shaft 7 with a smooth surface in the prior art, in this application example, the hub 9 is bound with the groove 17, which increases the binding force between the impeller 8 and the impeller rotating shaft 7. At the same time, because the connecting part is the groove 17, the area where the connecting part is located can have a certain bending deflection.

In this application example, the impeller 8 is made of a polymer material, preferably one of a natural rubber, an isoprene rubber or thermoplastic polyurethane.

In this application example, the impeller rotating shaft 7 is made of a metal material, preferably one of a cobalt-chromium alloy, a nickel-titanium alloy, a stainless steel (304 stainless steel or 316 stainless steel) or a tungsten alloy. The impeller rotating shaft 7 can be hardened by DLC or surface nitriding.

In this application example, the connecting part is provided, so that the binding force between the impeller rotating shaft 7 and the impeller 8 is improved, the impeller 8 is prevented from moving with respect to the impeller rotating shaft 7, the impeller 8 and the impeller rotating shaft 7 are prevented from being out of rotation, the working efficiency of the impeller 8 is improved, the risk that the impeller 8 scrapes with the housing 4 is avoided, hemolysis is reduced, and particles entering the body are reduced.

In this application example, the distal end of the impeller rotating shaft 7 is rotatably connected with the proximal end of the pigtail pipe 1 through a rotating structure 15. The rotating structure 15 can be of two types. As shown in FIG. 7, the first rotating structure 15 is a shaft sleeve 73. The shaft sleeve 73 is made of ceramics. The distal end of the impeller rotating shaft 7 is located at the inner side of the shaft sleeve 73 and is fixed with the shaft sleeve 73. As shown in FIG. 6, the second rotating structure 15 is a bearing, preferably a ball bearing. The distal end of the impeller rotating shaft 7 is located at the inner side of the inner ring 67 of the bearing and is fixed with the inner ring 67 of the bearing. The pigtail pipe 1 is located at an outer side of the outer ring 68 of the bearing and is fixed with the outer ring 68 of the bearing. If the impeller rotating shaft 7 is fixed with the inner ring 67 of the bearing, the impeller rotating shaft 7 is axially fixed with respect to the pigtail pipe 1. If the impeller rotating shaft 7 is matched with the shaft sleeve 73, the impeller rotating shaft 7 can move axially with respect to the pigtail pipe 1. The distal end of the impeller rotating shaft 7 is provided with a rotating shaft step 16. The proximal end of the rotating structure 15 is matched with the rotating shaft step 16 to axially limit the impeller rotating shaft 7 and restrict the impeller rotating shaft 7 from moving to the distal end of the catheter pump.

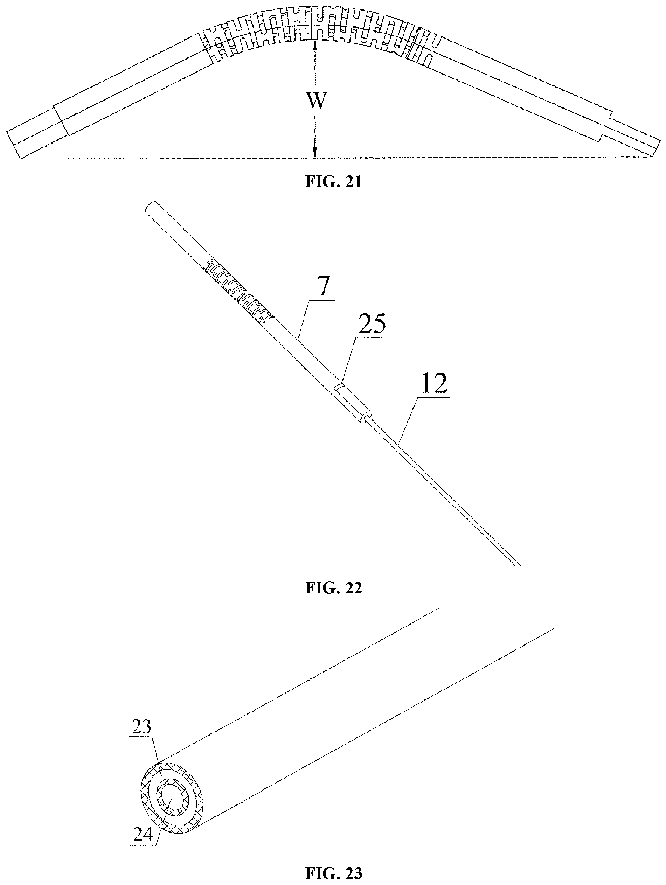

In this application example, as shown in FIG. 23, the multi-cavity pipe 5 is made of a plastic material such as polyamide and Pebax. The multi-cavity pipe 5 includes an inner pipe 26 and an outer pipe 27. The inner pipe 26 is coaxially provided at an inner side of the outer pipe 27. The perfusion cavity 23 is provided between the inner pipe 26 and the outer pipe 27. The transmission shaft 12 is coaxially provided in the inner pipe 26. The outflow cavity 24 is provided between the transmission shaft 12 and the inner pipe 26. The transmission shaft 12 is configured to transmit a driving torque. The transmission shaft 12 is made of a stainless steel or a Co-Cr-Mo alloy. A spring pipe 28 is provided between the transmission shaft 12 and the inner pipe 26. The outer wall of the spring pipe 28 is in contact with or not in contact with the inner wall of the inner pipe 26. The spring pipe 28 is made of a stainless steel. The spring pipe 28 is used to restrain the radial runout of the transmission shaft 12 and reduce the running vibration and the noise. The perfusion cavity 23 extends from the proximal end of the multi-cavity pipe 5 to the distal end of the multi-cavity pipe 5. The outflow cavity 24 extends from the proximal end of the multi-cavity pipe 5 to the distal end of the multi-cavity pipe 5. The distal end of the outflow cavity 24 is communicated with the distal end of the perfusion cavity 23. The proximal end of the impeller rotating shaft 7 is connected with the distal end of the transmission shaft 12. As shown in FIG. 22, the impeller rotating shaft 7 is provided with an inlet 25 communicated with the liquid path channel 11. The inlet 25 is a through-hole structure provided on the side wall of the impeller rotating shaft 7. The inlet 25 is communicated with the perfusion cavity 23, the liquid path channel 11 and the outflow cavity 24, respectively. Perfusion fluid flows from the perfusion cavity 23, flows into the liquid path channel 11 from the proximal end of the impeller rotating shaft 7 through the inlet 25, flows along the liquid path channel 11 to the distal end of the impeller rotating shaft 7, flows out from a gap between the impeller rotating shaft 7 and the rotating structure 15, and then enters the human body. At the same time, the perfusion fluid flows out from the outflow cavity 24. Perfusion liquid flows in the catheter pump, which can increase the interface lubrication, reduce the friction, and cool down and dissipate heat.

In this application example, a sheath cover 34 is provided at an outer side of a joint between the proximal end of the impeller rotating shaft 7 and the distal end of the transmission shaft 12. The sheath cover 34 can be made of the same material as the impeller rotating shaft 7 or the ceramic material. There is a gap formed between the impeller rotating shaft 7 and the inner side of the sheath cover 34. The fit gap is 1-8 um, preferably 1-3 um, so that the impeller rotating shaft 7 can rotate with respect to the sheath cover 34. Both ends of the sheath cover 34 are connected with the expandable bracket 3 and the multi-cavity pipe 5, respectively. The sheath cover 34 is provided with an opening 35. The opening 35 enables the distal end of the perfusion cavity 23 to be communicated with the distal end of the outflow cavity 24.

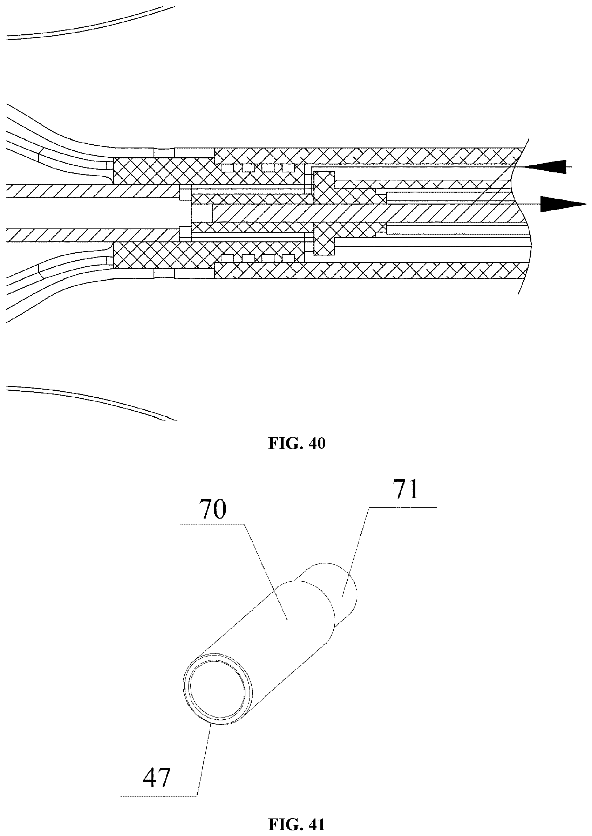

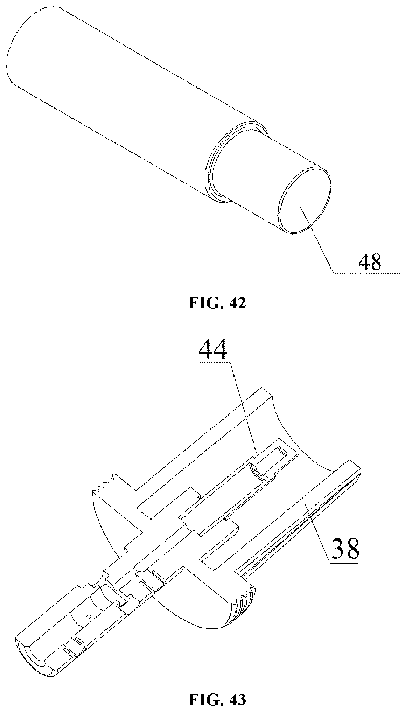

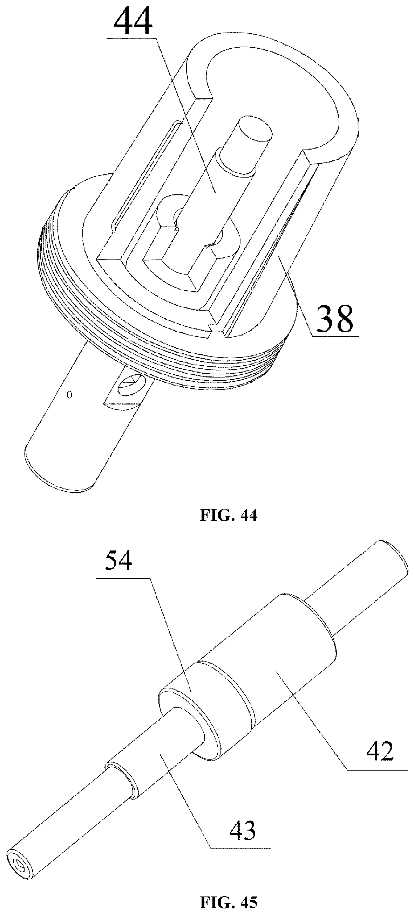

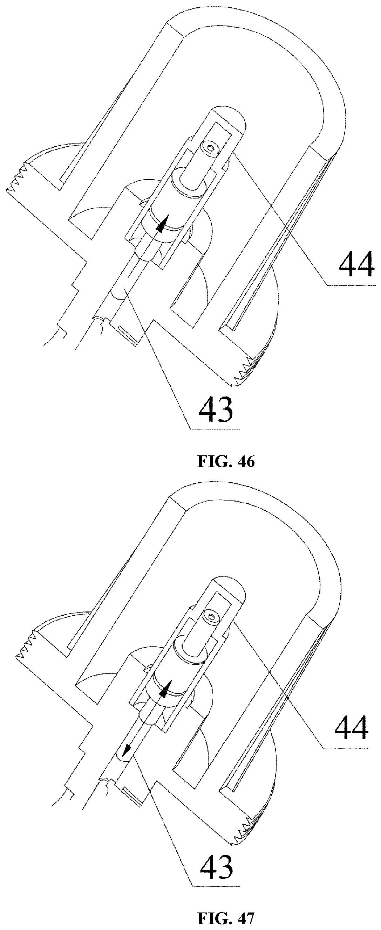

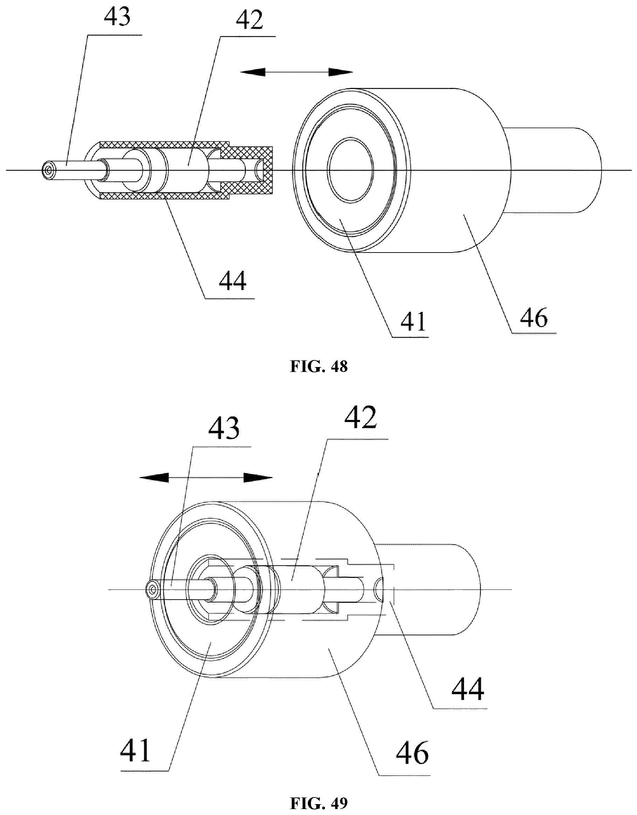

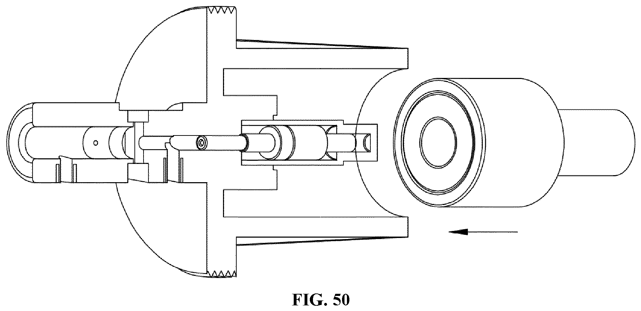

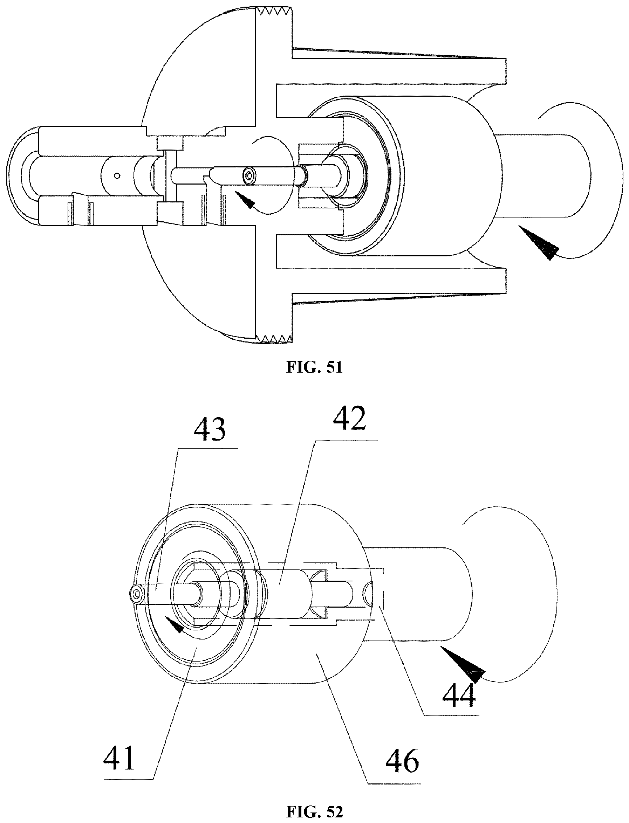

In this application example, as shown in FIG. 41 to FIG. 52, the handle module 6 includes a perfusion chamber 38, a transmission sealing structure, a driving motor 40 and a proximal end insert 45. The perfusion cavity 23 of the multi-cavity pipe 5 is communicated with the perfusion port of the perfusion chamber 38. The outflow cavity 24 of the multi-cavity pipe 5 is communicated with the outflow port of the perfusion chamber 38. The proximal end insert 45 is made of a metal material such as a stainless steel. The proximal end insert 45 is sleeved at the outer side of the transmission shaft 12, and there is a gap between the proximal end insert 45 and the transmission shaft 12. The proximal end of the proximal end insert 45 is fixedly connected with the perfusion chamber 38. The distal end of the proximal end insert 45 is hermetically connected with the inner cavity of the multi-cavity pipe 5. The proximal end insert 45 plays a role in separating the outflow cavity 24 from the perfusion cavity 23, so that the proximal end of the multi-cavity pipe 5 is connected with the perfusion chamber 38, and the proximal end of the perfusion cavity 23 is not communicated with the proximal end of the outflow cavity 24. The transmission sealing structure includes an external magnetic component 41, an internal magnetic component 42, a driven shaft 43 and a sealing cover 44. The external magnetic component 41, the internal magnetic component 42, the driven shaft 43 and the sealing cover 44 are all coaxially provided. Both the internal magnetic component 42 and the external magnetic component 41 are made of neodymium iron boron and other materials. Each of the internal magnetic component 42 and the external magnetic component 41 includes a magnetic ring. The internal magnetic component 42 is sleeved on the driven shaft 43 and is adhered to the driven shaft 43. The internal magnetic component 42 is configured to receive a magnetic torque. The sealing cover 44 is provided at an outer side of the internal magnetic component 42. The external magnetic component 41 is provided on an external magnetic component mounting sleeve 46. The external magnetic component mounting sleeve 46 is located at the inner side of the perfusion chamber 38. The external magnetic component mounting sleeve 46 is fixedly connected with the external magnetic ring and the output shaft of the driving motor 40, respectively. The external magnetic component mounting sleeve 46 is configured to transmit the rotating torque of the output shaft of the driving motor 40 and fix the external magnetic ring. The external magnetic component mounting sleeve 46 is made of engineering plastics such as Peek, PC, ABC, etc. The external magnetic component 41 is located at an outer side of the sealing cover 44. There is a gap formed between the external magnetic component 41 and the sealing cover 44. The sealing cover 44 is made of non-metallic materials, such as ceramics (such as alumina and zirconia), glass, diamond and other materials. Alternatively, the sealing cover 44 is made of plastic, and a non-metallic wear-resistant coating is provided outside the plastic, so that the opening end 47 of the sealing cover 44 is hermetically connected with the perfusion chamber 38 by adhesion. The outer wall of the sealing cover 44 is hermetically connected with the perfusion chamber 38 through interference fit. The sealing cover 44 is configured to isolate the internal magnetic component 42 from the external magnetic component 41. The external magnetic component 41 rotates, which in turn drives both the internal magnetic component 42 and the driven shaft 43 to rotate with respect to the sealing cover 44. The driven shaft 43 and the internal magnetic component 42 can also move in the axial direction of the sealing cover 44.

In this application example, the handle module 6 further includes a first diffusion stress pipe 55, a motor housing 56, a second diffusion stress pipe 57 and a cable 58. The first diffusion stress pipe 55 is made of elastic materials such as rubbers, silica gel or polyurethane. The distal end of the first diffusion stress pipe 55 is fixedly connected with the multi-cavity pipe 5. The proximal end of the first diffusion stress pipe 55 is fixedly connected with the perfusion chamber 38. The first diffusion stress pipe 55 is configured to reduce the stress concentration between the multi-cavity pipe 5 and the perfusion chamber 38, and reduce the folding. The first diffusion stress pipe 55 is provided with a perfusion pipe 59 and an outflow pipe 60. The perfusion pipe 59 is made of materials such as PVC and polyurethane. One end of the perfusion pipe 59 is communicated with the perfusion port. The perfusion pipe 59 is configured to deliver perfusion liquid (heparin saline) into the body. The outflow pipe 60 is made of materials such as PVC and polyurethane. One end of the outflow pipe 60 is communicated with the outflow port. The outflow pipe 60 is configured to receive liquid refluxed in the body and allow the liquid to flow back to the waste liquid bag outside the body. The motor housing 56 is provided at an outer side of the driving motor 40. The motor housing 56 is detachably connected with the perfusion chamber 38, and the motor housing 56 is connected with the perfusion chamber 38 through a locking nut 61. The locking nut 61 is made of ABS/PC. The second diffusion stress pipe 57 is located at an outer side of the driving motor 40 and at an inner side of the housing of the driving motor 40. The distal end of the second diffusion stress pipe 57 is connected with the motor housing 56. The proximal end of the second diffusion stress pipe 57 is connected with the cable 58. One end of the cable 58 is connected with the drive motor 40. The cable 58 is configured to provide power to the motor and transmit the control and detection signals. The second diffusion stress pipe 57 is made of elastic materials such as rubbers, silica gel or polyurethane. The second diffusion stress pipe 57 is configured to reduce the running vibration and the noise of the motor.

In this application example, the sealing cover 44 is integrally molded. The sealing cover 44 includes a first connecting part 70 and a second connecting part 71. One end of the first connecting part 70 is an opening end 47, and the other end of the first connecting part 70 is connected with one end of the second connecting part 71. The other end of the second connecting part 71 is a closed end. The driven shaft 43 is provided with a projection 54. The projection 54 is rotatably connected with the first connecting part 70 of the sealing cover 44. The second connecting part 71 of the sealing cover 44 is rotatably connected with the driven shaft 43.

In this application example, the driven shaft 43 has a solid structure. The solid structure is formed by winding multiple steel cables. The distal end of the driven shaft 43 is connected with the proximal end of the transmission shaft 12. Alternatively, the driven shaft 43 has a hollow structure. The hollow structure is a hollow torque spring formed by weaving multiple steel cables. The proximal end of the transmission shaft 12 extends into the driven shaft 43. The transmission shaft 12 is a solid shaft.

In this application example, the distal end of the driven shaft 43 protrudes from the opening end 47 of the sealing cover 44, and there is a gap formed between the driven shaft 43 and the perfusion chamber 38. The gap formed between the driven shaft 43 and the perfusion chamber 38 can be communicated with the outflow cavity 24. The proximal end of the driven shaft 43 is close to the sealing end 48 of the sealing cover 44. There is gap formed between the driven shaft 43 and the sealing cover 44, and there is gap formed between the internal magnetic component 42 and the sealing cover 44.

When the transmission sealing structure of this application example is used, the driving motor 40 drives the external magnetic component mounting sleeve 46 to drive the external magnetic component 41 to rotate. The external magnetic component 41 drives the internal magnetic component 42 and the driven shaft 43 to rotate Through the magnetic cooperation between the external magnetic component 41 and the internal magnetic component 42. The driven shaft 43 drives the transmission shaft 12 to rotate. The transmission shaft 12 drives the impeller rotating shaft 7 and the impeller 8 to rotate.

The sealing cover 44 of this application example not only can achieve the sealing function, but also achieve the rotatable connection, so as to achieve the integrated design of sealing and rotatable connection. In the transmission process, the external magnetic component 41 is not in contact with the internal magnetic component 42. Moreover, the driven shaft 43 not only can rotate, but also move in the axial direction of the driven shaft 43. The transmission of this application example is simple in sealing structure, high in reliability, long in service life and low in cost.

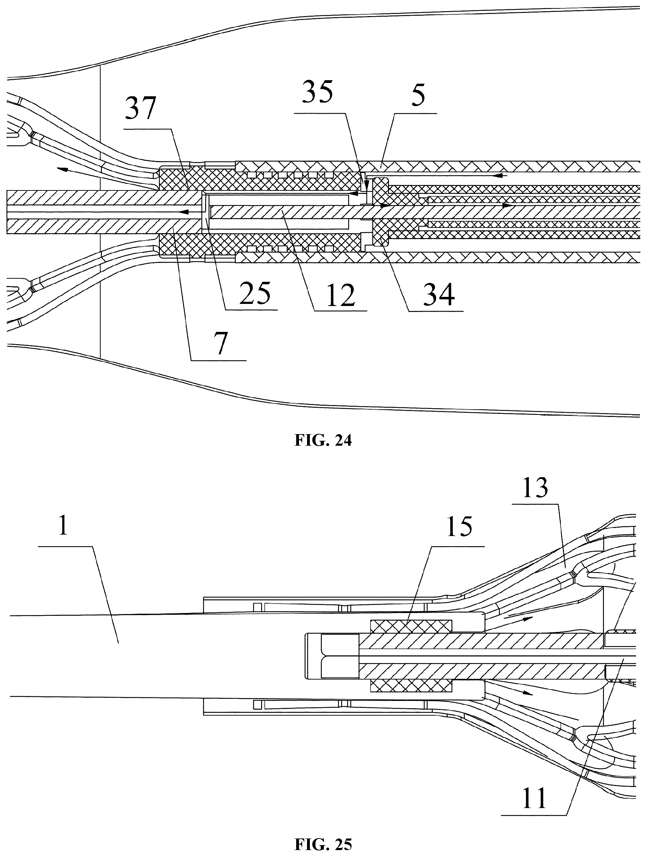

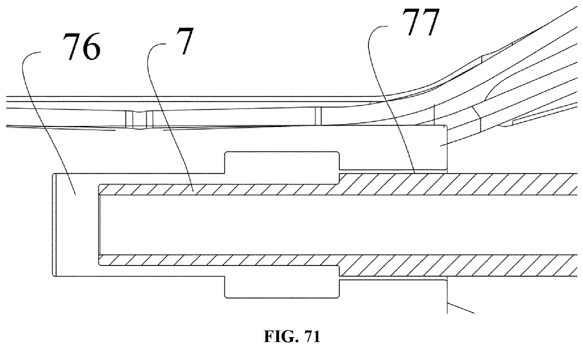

In this application example, as shown in FIG. 24, FIG. 70, FIG. 71 and FIG. 53, perfusion liquid enters the perfusion cavity 23 through the perfusion pipe 59 and is divided into two parts. One part of the perfusion liquid enters the liquid path channel 11 through the inlet 25, flows to the distal end of the impeller rotating shaft 7, flows out from the gap between the impeller rotating shaft 7 and the rotating structure 15, and then enters the human body. The other part of the perfusion liquid flows out of the outflow cavity 24 and then is divided into two parts, in which one part flows into the waste liquid bag through the outflow pipe 60, and the other part flows to the space formed by the driven shaft 43, the internal magnetic component 42 and the sealing cover 44 from the gap between the driven shaft 43 and the perfusion chamber 38, flows out from the gap between the driven shaft 43 and the perfusion chamber 38, and then is discharged through the outflow pipe 60.

### Application Example 2

The difference between this application example and Application Example 1 is that, as shown in FIG. 25, in this application example, there is no rotating shaft step 16 at the distal end of the impeller rotating shaft 7, and the sheath cover 34 doesn't need to be provided with the sheath cover limiting step 36. The proximal end of the impeller rotating shaft 7 is fixedly connected with the distal end of the transmission shaft 12. When the expandable bracket 3 changes from the contracted state to the expanded state, the distal end of the expandable bracket 3 drives the pigtail pipe 1, which in turn drives the rotating structure 15 to move to the proximal end with respect to the impeller rotating shaft 7. At this time, the sheath cover limiting step 36 does not play a limiting role. Because the impeller rotating shaft 7 is fixedly connected with the transmission shaft 12, and the proximal end of the transmission shaft 12 is fixedly connected with the driven shaft 43, the sealing end 48 of the sealing cover 44 restricts the proximal end of the driven shaft 43, thereby restricting the impeller rotating shaft 7 from moving excessively to the proximal end.

### Application Example 3

In order to solve the problem that the impeller rotating shaft 7 cannot be bent in the prior art, in this application example, the impeller rotating shaft 7 includes a first rigid section 20, a flexible section 21 and a second rigid section 22 which are sequentially provided. The impeller 8 and the connecting part are both located in the flexible section 21, the connecting part is a through hole 18, and the flexible section 21 is bendable.

In this application example, the flexible section 21 has a hypotube structure. The hypotube structure can use the hypotube structure in the prior art. The flexible section 21 of this application example enables the impeller structure 2 to bendable in the axial direction, which reduces the damage of the blood pumping catheter to the blood vessel wall in the pushing process, and solves the problem that the existing impeller rotating shaft 7 cannot be bent.

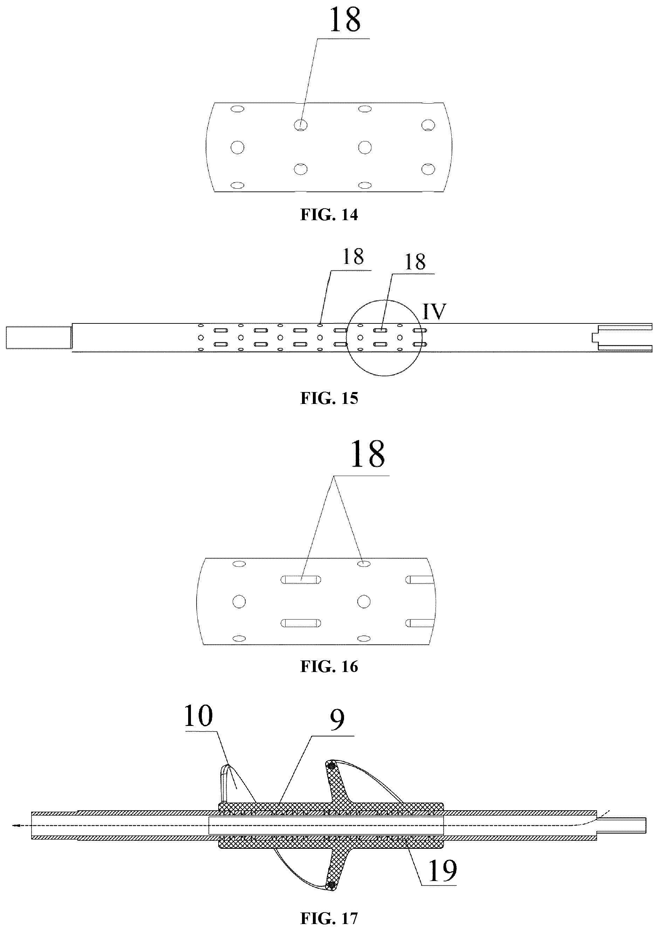

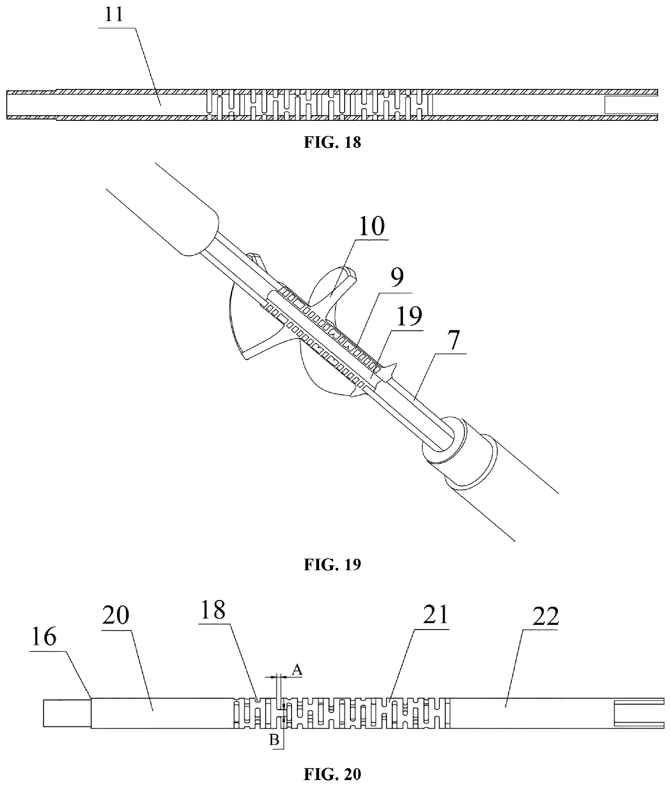

As shown in FIG. 17 and FIG. 19, in this application example, the liquid path channel 11 is communicated with the through hole 18. The hub 9 passes through the through hole 18 and forms a film layer 19 on an inner wall of the impeller rotating shaft 7, so that the bonding force between the impeller 8 and the impeller rotating shaft 7 is further improved. The hub 9 covers the inner wall and the outer wall of the impeller rotating shaft 7 where the through hole 18 is located. The part of the hub 9 on the inner wall of the impeller rotating shaft 7 is in contact with the inner wall of the impeller rotating shaft 7. The part of the hub 9 on the outer wall of the impeller rotating shaft 7 is in contact with the outer wall of the impeller rotating shaft 7. The part of the hub 9 in the through hole 18 is in contact with the side wall of the through hole 18 to seal the through hole 18 and prevent the perfusion liquid from leaking from the through hole. The through hole 18 is provided, which further improves the bending deflection of the impeller rotating shaft 7.

When the impeller rotating shaft 7 and the impeller 8 are integrally injection molded, first, the cut impeller rotating shaft 7 is placed in the mold cavity for positioning. Second, a cylindrical and stepped shaft center extends into the liquid path channel 11 from both ends of the impeller rotating shaft 7. The gap between the shaft center and the inner wall of the impeller rotating shaft 7 is configured to form the part of the hub 9 on the inner wall of the impeller rotating shaft 7, that is, the film layer 19. Finally, the mold cavity is removed to obtain the impeller structure 2.

### Application Example 4

This application example defines the specific structure of the hypotube structure in Application Example 3. As shown in FIG. 18, in this application example, the side wall of the flexible section 21 is provided with several circles of through-hole structures in the axial direction of the flexible section 21. The through-hole structures of each circle includes several through-holes 18 provided in the circumferential direction of the flexible section 21. The through-holes 18 are discontinuous in the circumferential direction of the flexible section 21 so as to maintain the torque transmission of the impeller rotating shaft 71. The through-holes 18 of adjacent circles are staggered, and the shape of the through-holes 18 is not limited.

### Application Example 5

As shown in FIG. 20 and FIG. 21, this application example further defines Application Example 4. In this application example, the through holes 18 are long holes. The size B of the connecting part between adjacent through holes 18 of the through-hole structure of the same circle is 0.05-2 mm, preferably 0.1-0.5 mm, more preferably 0.25 m. The size A of the through hole 18 in the axial direction of the flexible section 21 is 0.1-0.5 mm, preferably 0.1-0.5 mm, and more preferably 0.25 mm. In this application example, the maximum bending deflection W of the impeller rotating shaft 7 is in the range of 0.1-5 mm.

In this application example, the existence of the through hole 18 not only can improve the binding force between the impeller 8 and the impeller rotating shaft 7, but also increase the bending deflection of the impeller rotating shaft 7.

### Application Example 6

As shown in FIG. 11 to FIG. 12, in this application example, the through holes 18 of adjacent circles are provided in one-to-one correspondence, and the through holes 18 are round holes.

### Application Example 7

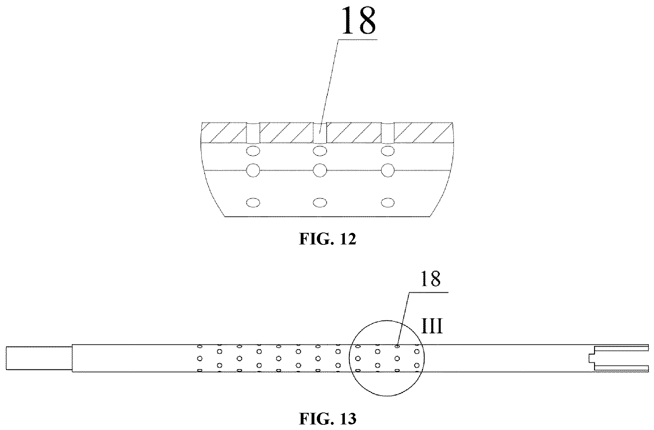

As shown in FIG. 13 to FIG. 14, the difference between this application example and Application Example 6 is that in this application example, the through holes 18 are round holes, and the through holes 18 of adjacent circles are staggered.

### Application Example 8

As shown in FIG. 15 to FIG. 16, in this application example, the through holes 18 include round holes and long holes. Each through hole 18 of the through-hole structure of the same circle is a round hole. Each through hole 18 of the through-hole structure adjacent to the round hole of the through-hole structure is a long hole, and the through holes 18 of the adjacent circles are staggered.

### Application Example 9

The difference between this application example and Application Example 1 is that in this application example, the connecting part is a groove 17 and a through hole 18.

### Application Example 10

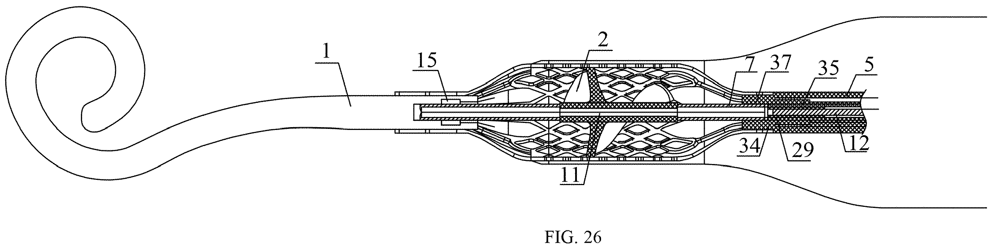

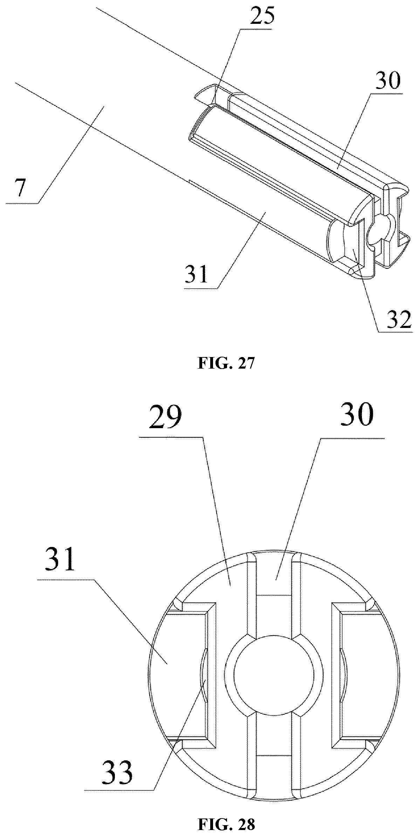

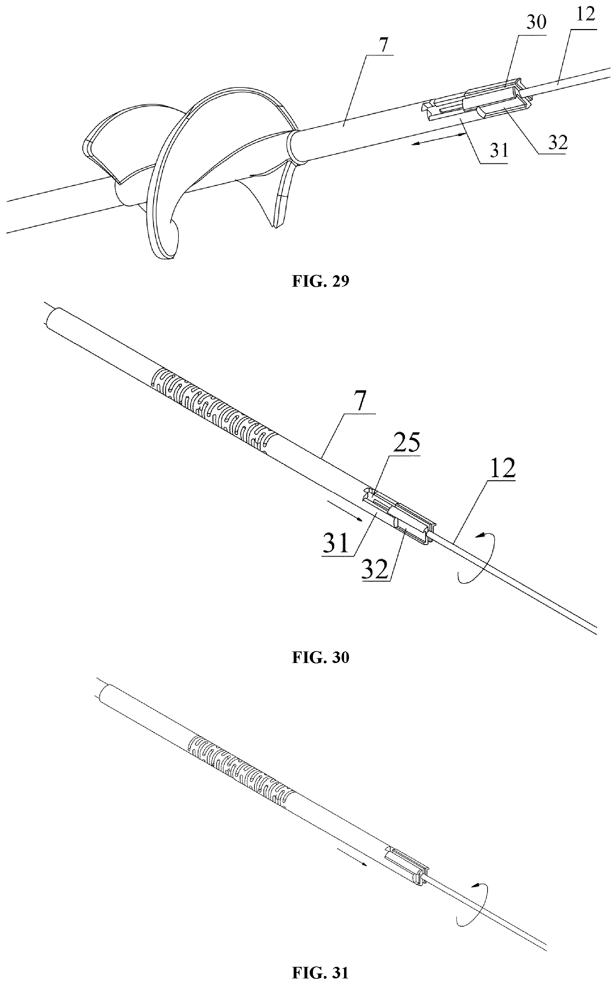

As shown in FIG. 26 to FIG. 31, this application example is further optimized on the basis of Application Example 1. This application example includes a switching block 29. The switching block 29 can be made of the same material as the impeller rotating shaft 7 or the ceramic material. The proximal end of the impeller rotating shaft 7 is connected with the distal end of the transmission shaft 12 through the switching block 29. In this application example, the proximal end of the impeller rotating shaft 7 is provided with protrusions 31. The inlet 25 is provided between adjacent protrusions 31. The inlet 25 has a notched structure. The switching block 29 is provided with a first channel 30. The first channel 30 is communicated with the inlet 25, the perfusion cavity 23 and the outflow cavity 24, respectively.

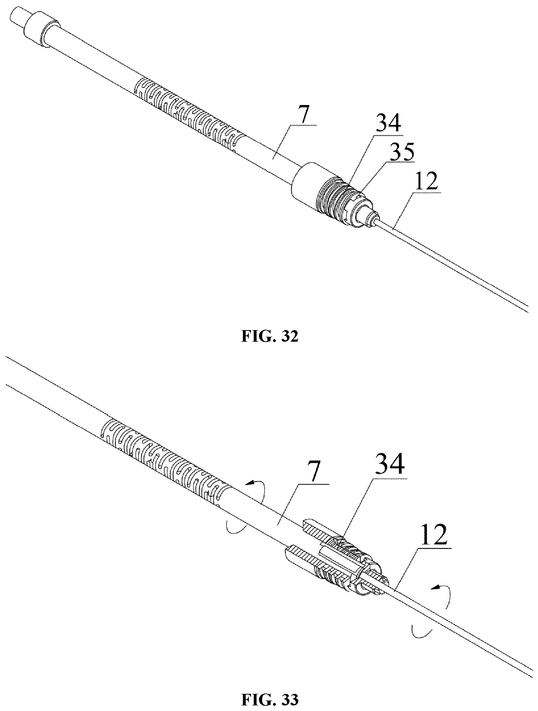

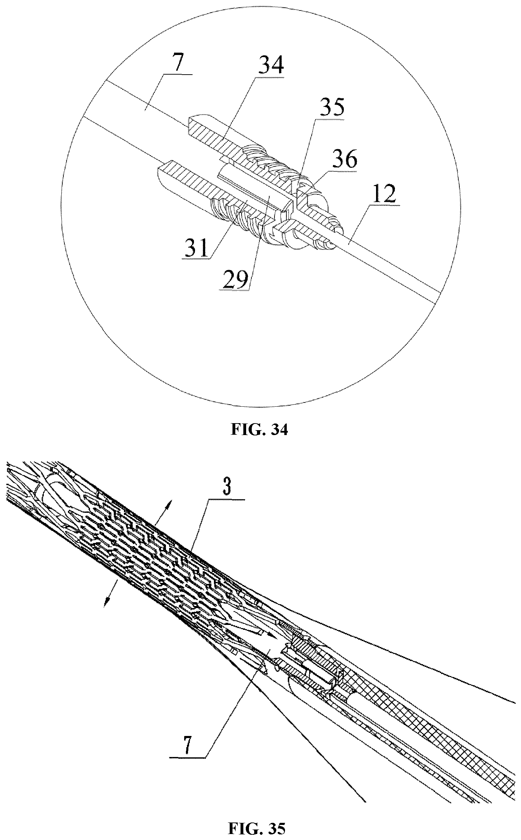

In this application example, as shown in FIG. 32 to FIG. 36, a sheath cover 34 is provided at the outer side of the joint of the proximal end of the impeller rotating shaft 7 and the switching block 29. There is a gap between the outer side of the joint of the proximal end of the impeller rotating shaft 7 and the switching block 29 and the inner side of the sheath cover 34. The fit clearance of the gap is 1-8 um, preferably 1-3 um, so that the joint of the proximal end of the impeller rotating shaft 7 and the switching block 29 is rotatable with respect to the sheath cover 34. The sheath cover 34 is provided with a sheath cover limiting step 36. The impeller rotating shaft 7 is provided with a rotating shaft step 16. The proximal end of the impeller rotating shaft 7 is in axial sliding fit with the switching block 29. The switching block 29 is fixedly connected with the transmission shaft 12. When the expandable bracket 3 changes from the contracted state to the expanded state, the distal end of the expandable bracket 3 drives the pigtail pipe 1, which in turn drives the rotating structure 15. The rotating structure 15 drives the impeller rotating shaft 7. The impeller rotating shaft 7 drives the switching block 29 to move to the proximal end. The sheath cover limiting step 36 restricts the switching block 29 from moving excessively to the proximal end.

In this application example, the switching block 29 is provided with a chute 32. The protrusion 31 is in sliding connection with the chute 32. The gap between the inner surface of the protrusion 31 and the surface of the chute 32 is 0.1-0.5 mm. A second channel 33 is formed between the inner surface of the protrusion 31 and the surface of the chute 32. The second channel 33 is communicated with the perfusion cavity 23, the liquid path channel 11 and the outflow cavity 24, respectively.

In this application example, the perfusion liquid is divided into three parts. After perfusion liquid enters from the perfusion cavity 23, a part of the perfusion liquid enters the liquid path channel 11 through the first channel 30 and the inlet 25. At the same time, a part of the perfusion liquid enters the liquid path channel 11 through the second channel 33, and the perfusion liquid flows to the distal end of the impeller rotating shaft 7, flows out from the gap between the impeller rotating shaft 7 and the rotating structure 15, and then enters the human body. Another part of the perfusion liquid flows out from the outflow cavity 24.

### Application Example 11

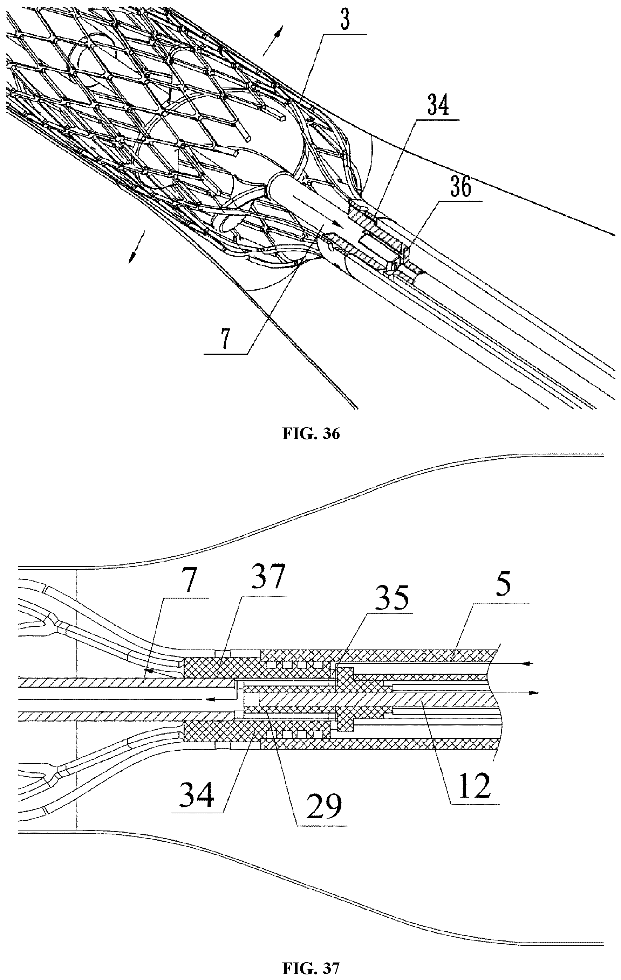

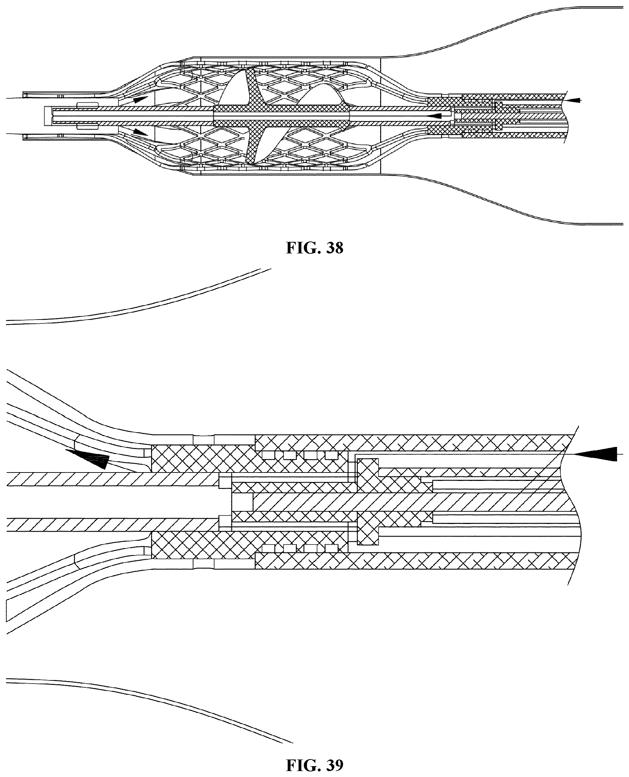

As shown in FIG. 37 to FIG. 40, this application example is further optimized on the basis of Application Example 10. A sheath cover 34 is provided at the outer side of the joint of the proximal end of the impeller rotating shaft 7 and the switching block 29. The sheath cover 34 is hermetically connected with the inner pipe 26 and the outer pipe 27 of the multi-cavity pipe 5, respectively.

In this application example, there is a third channel 37 between the outer wall of the proximal end of the impeller rotating shaft 7 and the inner wall of the sheath cover 34. The size of the third channel 37 is 0.003-0.2 mm. The proximal end of the third channel 37 is communicated with the distal ends of the perfusion cavity 23, the first channel 30, the second channel 33 and the outflow cavity 24, respectively. The distal end of the third channel 37 is communicated with the human body.

In this application example, the sheath cover 34 is provided with an opening 35, so that the first channel 30, the second channel 33, the third channel 37, the perfusion cavity 23 and the outflow cavity 24 can be communicated with each other.

In this application example, the perfusion liquid is divided into four parts. After the perfusion liquid enters from the perfusion cavity 23, a part of the perfusion liquid enters the liquid path channel 11 through the first channel 30 and the inlet 25. At the same time, a part of the perfusion liquid enters the liquid path channel 11 through the second channel 33, and the perfusion liquid flows to the distal end of the impeller rotating shaft 7, flows out from the gap between the impeller rotating shaft 7 and the rotating structure 15, and then enters the human body. A part of the perfusion fluid enters the human body through the third channel 37. Another part of the perfusion liquid flows out from the outflow cavity 24.

### Application Example 12

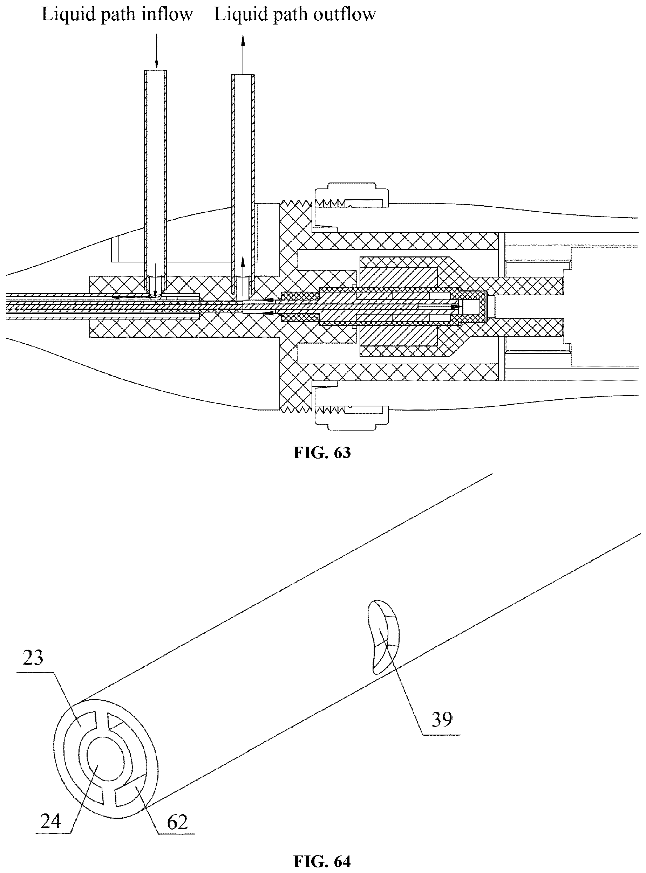

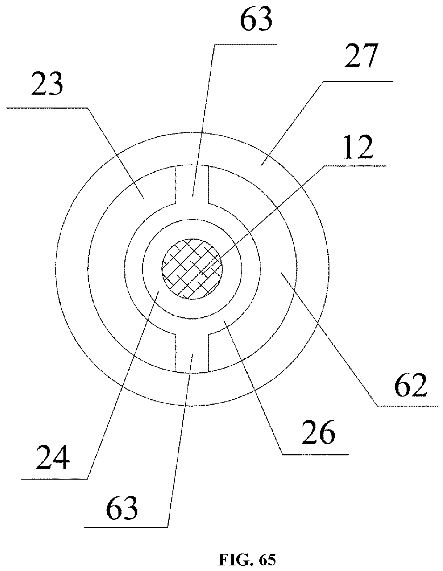

As shown in FIG. 64 to FIG. 65, the difference between this application example and Application Example 1 is that in this application example, a pressure measuring cavity 62 is further provided between the inner pipe 26 and the outer pipe 27. The pressure measuring cavity 62 is separated from the perfusion cavity 23 by a partition plate 63. The pressure measuring cavity 62 is not communicated with the perfusion cavity 23. The pressure measuring cavity 62 is communicated with the human body through the pressure measuring hole 39 provided on the side wall of the outer pipe 27. The front end of the pressure measuring hole 39 is provided with a plug 72 to prevent the pressure measuring cavity 62 from being communicated with the perfusion cavity 23 and the outflow cavity 24. A detection cavity 64 is provided in the perfusion chamber 38. The detection cavity 64 is communicated with the pressure measuring cavity 62 and a pressure measuring pipe 65 through which pressure measuring liquid passes, respectively. A pressure measuring element 66 is provided in the detection cavity 64. The pressure measuring element 66 is preferably a pressure sensor. The pressure measuring element 66 is configured to detect an internal pressure conducted by the pressure measuring liquid.

Since the perfusion chamber 38 is detachably connected with the motor housing 56, the transmission line 74 of the pressure measuring element 66 is provided with a transmission line antenna 75 at a corresponding position.

### Application Example 13

As shown in FIG. 54 to FIG. 55, the difference between this application example and Application Example 1 is that in this application example, each of the internal magnetic component 42 and the external magnetic component 41 includes at least two magnetic rings. The magnetic rings in the internal magnetic component 42 are provided in the axial direction of the driven shaft 43. The magnetic rings in the external magnetic component 41 are provided in the axial direction of the driven shaft 43. By increasing the volume of the internal magnetic component 42 and the external magnetic component 41 (for example, increasing the length, the thickness, the surface area, etc.), the strength of the internal magnetic component 42 and the external magnetic component 41 is increased, thereby increasing the magnetic torque.

### Application Example 14

As shown in FIG. 56 to FIG. 59, the difference between this application example and Application Example 13 is that in this application example, the driven shaft 43 includes a first shaft part 50 and a second shaft part 51. The first shaft part 50 is located inside the sealing cover 44. The second shaft part 51 is located outside the sealing cover 44. A spiral structure 52 is provided at an outer side of the second shaft part 51. A spiral direction of the spiral structure 52 is opposite to a rotation direction of the driven shaft 43. For example, if the driven shaft 43 rotates to the right, the spiral direction of spiral structure 52 is left-handed. The spiral structure 52 is provided, so that the liquid in the space formed by the driven shaft 43, the internal magnetic component 42 and the sealing cover 44 can be promoted to flow out, the friction heat and the wear particles can be taken away, blood is prevented from existing in the space formed by the driven shaft 43, the internal magnetic component 42 and the sealing cover 44, and the protein in the blood is prevented from solidifying to hinder the rotation of the driven shaft 43 and the internal magnetic component 42.

### Application Example 15

As shown in FIG. 60 to FIG. 61, the difference between this application example and Application Example 14 is that in this application example, the transmission shaft 12 is a hollow shaft. That is, a return channel is provided inside the transmission shaft 12, and the return channel is communicated with the outflow cavity 24. Liquid is divided into two parts. One part flows to the space formed by the driven shaft 43, the internal magnetic component 42 and the sealing cover 44 from the gap between the driven shaft 43 and the perfusion chamber 38, flows out from the gap between the driven shaft 43 and the perfusion chamber 38, takes away the friction heat between the driven shaft 43 and the sealing cover 44 and then flows out through the outflow pipe 60. The other part flows to the space formed by the driven shaft 43, the internal magnetic component 42 and the sealing cover 44 through the return channel of the transmission shaft 12, and then flows out from the gap between the driven shaft 43 and the perfusion chamber 38.

### Application Example 16

The difference between this application example and Application Example 1 is that in this application example, both the internal magnetic component 42 and the external magnetic component 41 include at least two magnetic rings. All of the magnetic rings in the internal magnetic component 42 are sequentially nested in the radial direction of the driven shaft 43. All of the magnetic rings in the external magnetic component 41 are sequentially nested in the radial direction of the driven shaft 43.

### Application Example 17

The difference between this application example and Application Example 1 is that in this application example, both the internal magnetic component 42 and the external magnetic component 41 include several magnetic strips uniformly provided in the circumferential direction of the driven shaft 43. The number of magnetic strips of the internal magnetic component 42 is the same as or different from that of the external magnetic component 41.

### Application Example 18

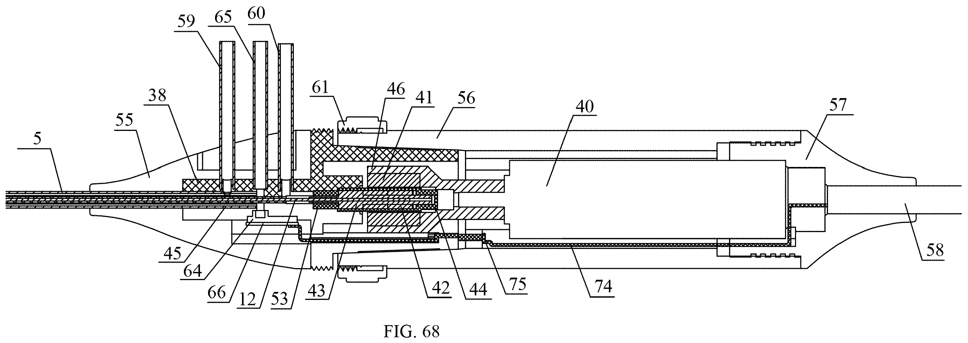

As shown in FIG. 62 to FIG. 69, the difference between this application example and Application Example 12 to Application Example 17 is that in this application example, a rotating shaft step 16 is not provided on the impeller rotating shaft 7. A wear-resistant insert 53 is provided between the driven shaft 43 and the perfusion chamber 38. The wear-resistant insert 53 is made of ceramic materials, such as alumina and zirconia. The wear-resistant insert 53 is fixedly connected with the perfusion chamber 38. When the driven shaft 43 rotates, the wear-resistant insert 53 forms bearing fit with the driven shaft 43 and is rotatable with respect to the driven shaft, and the wear-resistant insert 53 is matched with the projection 54 to axially limit the driven shaft 43 and restrict the driven shaft 43 from moving to the distal end of the catheter pump.

The impeller structure 2 of the present disclosure is bendable in the axial direction, and the damage of the catheter pump to the blood vessel wall in the pushing process is reduced. The impeller rotating shaft 7 is integrally injection molded with the impeller 8, and the binding force between the impeller 8 and the impeller rotating shaft 7 is increased through the connecting part on the impeller rotating shaft 7. The application example has a stable support, and the impeller rotating shaft 7 is a continuous shaft, thus avoiding the problem in the prior art that the impeller rotating shaft 7 uses a multi-section shaft to result in radial deflection, reducing the risk that the impeller 8 scrapes with the housing 4, reducing hemolysis and reducing particles entering the body. The application example is simple in structure, low in cost and high in reliability.

### Application Example 19

After in-depth and extensive research, the applicant found that most interventional instruments (such as intravascular blood pumping devices, intracardiac blood pumping devices, thrombus aspiration catheters, rotary ablation thrombolysis catheters, etc.) that are inserted by minimally invasive operation and transmit a torque in a human body through a flexible transmission shaft (such as a transmission shaft 12) or drive a functional unit at a distal end of a flexible transmission shaft (such as an impeller structure 2, a thrombolysis mesh basket, a rotary ablation blade, etc.) to rotate (such as driving the impeller 8 to rotate) are troubled by the technical problem that the functional unit and the fixed rotating shaft thereof (such as the impeller rotating shaft 7 fixed with the impeller 8 on the impeller structure 2) precipitate particles into the body/vascular blood due to wear after interacting with other structures (such as the pigtail pipe 1, the sheath cover 34 and the expandable bracket 3) under working conditions (such as interface friction and unstable collision).

The above technical problems are particularly obvious in some interventional instruments that need to be provided with rotating shaft support components at both ends of or one end of the rotating shaft, and the functions of the rotating shaft support components include a radial support and a thrust support, which are difficult to solve. The rotating shaft support component includes a radial support and an axial thrust support. The rotating shaft is configured to pass through the shaft center of a functional element (such as the impeller 8) and is fixedly connected with the functional element. The radial support is configured to reduce the radial movement of the functional unit by keeping the rotating shaft of the functional unit at a given radial position. The purpose of the axial thrust support is to resist the axial force of the functional unit and hinder the axial movement of the rotating shaft, so as to reduce or inhibit the axial position change of the functional unit. In some specific applications (such as axial-flow pumping blood), the rotating shaft of the functional unit must be equipped with an axial thrust support in addition to a radial support (such as an axial-flow pump, which is different from centrifugal pump in principle, where its blades are subjected to the force from the fluid in the working state, that is, the fluid pushes the blades to the direction opposite to its own movement direction). In some specific applications, the axial thrust support structure of the functional unit in the interventional instrument can be provided on the driving part outside the body (for example, in this application example, the impeller rotating shaft 7 is fixedly connected with the transmission shaft 12, and the proximal end of the transmission shaft 12 is fixedly connected with the driven shaft 43, so as to transfer the axial thrust support structure to the driving part outside the body) or on the rotating shaft of the functional unit inside the body (for example, in this application example, the distal end of the impeller rotating shaft 7 is provided with a rotating shaft step 16, and the proximal end of the rotating structure 15 is matched with the rotating shaft step 16 to axially limit the impeller rotating shaft 7 and restrict the impeller rotating shaft 7 from moving to the distal end of the catheter pump). However, when the axial thrust support structure is provided inside the body, particles are precipitated due to the interface friction and the wear, which is not expected.

In the existing liquid path design technology, lubrication of the axial thrust bearing interface is often neglected, so that particles precipitated due to the interface friction and the wear enter the body/vascular blood. However, in other existing technologies, the rotating shaft support component and the end of the rotating shaft are strengthened in wear resistance and hardened.

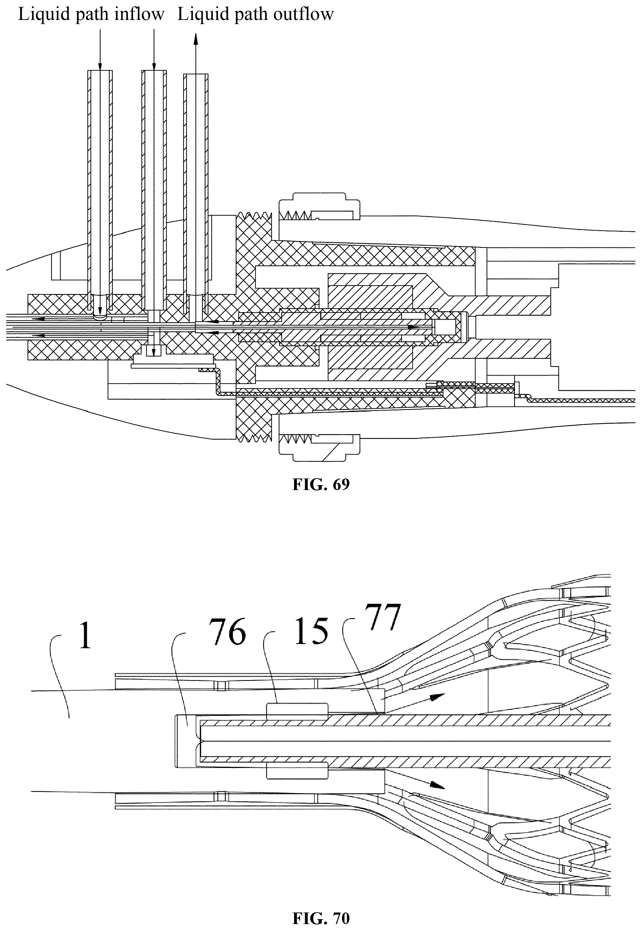

The present disclosure provides a method of inhibiting wear of a moving joint surface of a distal end section in an interventional instrument to precipitate particles. The core idea of the method is that the rotating shaft is suspended by a hydraulic pressure, so that the rotating shaft and the rotating shaft support component are in a non-contact or near-contact state, thus reducing the contact area of the moving joint surface between the rotating shaft and the rotating shaft support component. The specific method is as follows: a hydraulic cavity (that is, the rotating shaft is in gap fit with the rotating shaft support component) is provided on the moving joint surface between the rotating shaft and the rotating shaft support component for filling liquid. The hydraulic cavity is provided with a liquid injection port and an outflow port. The liquid injection port and the outflow port are provided in the rotating shaft, or the rotating shaft support component, or the fit gap between the rotating shaft and the rotating shaft support component. Liquid (such as physiological saline, other medically acceptable perfusion liquid) is injected into the hydraulic cavity through the injection port, and the liquid overflows/seeps/flows out to the outflow port. The liquid pressure in the hydraulic cavity is adjusted, so that the rotating shaft is supported by the liquid in the hydraulic cavity in the radial direction and/or the axial direction and is separated from the rotating shaft support component. It can be understood that the method has been applied and practiced in Application Example 1 of the present disclosure. That is, the perfusion liquid enters the perfusion cavity 23 through the perfusion pipe 59 and is divided into two parts. A part of the perfusion liquid enters the liquid path channel 11 through the inlet 25, flows to the distal end of the impeller rotating shaft 7 (as shown in FIG. 70 to FIG. 71, the hydraulic cavity 76), flows out from the gap between the impeller rotating shaft 7 and the rotating structure 15 (as shown in FIG. 70 to FIG. 71, the rotating shaft fit gap 77), and then enters the human body. When the parameters such as the injection velocity/flow rate/pressure of perfusion fluid are adjusted outside the body, the hydraulic pressure in the hydraulic cavity can be affected, so as to achieve the desired suppression of the interface friction and the wear of the distal functional unit in the interventional instrument to precipitate particles; reduce or replace the role of the shaft sleeve or the bearing with the hydraulic support.

Further, the rotating shaft support component includes a radial support and an axial thrust support. A hydraulic cavity is provided on the moving joint surface of the radial support and the rotating shaft. A hydraulic cavity is provided on the moving joint surface of the axial thrust support and the rotating shaft. The two hydraulic cavities are communicated with each other. Further, the injection port is provided at the end face or the side surface of the rotating shaft, and the outflow port is provided at the fit gap between the radial support and the rotating shaft (that is, the fit gap between the radial support and the rotating shaft serves as the outflow port). Further, the hydraulic pressure in the hydraulic cavity is greater than the hydraulic pressure at the outer side of the outflow port.

Based on the above method of inhibiting wear of the moving joint surface of the distal end section in the interventional instrument to precipitate particles, the applicant further provides:
an interventional instrument, which includes a functional unit for rotation operation, a flexible transmission shaft, a catheter and a handle structure, where the functional unit includes a rotating shaft and a functional element fixedly connected with the rotating shaft; the catheter is slender, the proximal end of the catheter is connected with the handle structure, and the distal end of the catheter is provided with a rotating shaft support structure which can provide a radial support and an axial thrust support to the rotating shaft; the flexible transmission shaft passes through the conduit, the distal end of the flexible transmission shaft is connected with the rotating shaft, and the proximal end of the flexible transmission shaft is connected with the handle structure to transmit the torque output by the handle structure to the rotating shaft; the functional unit can perform limited axial displacement with respect to the rotating shaft support structure. A hydraulic cavity is provided on the moving joint surface between the rotating shaft support structure and the rotating shaft, so that the rotating shaft can be supported by liquid in the radial direction and/or the axial direction to be separated from the moving joint surface of the rotating shaft support structure. The hydraulic cavity is provided with a liquid injection port and an outflow port.

Further, the functional unit of the interventional instrument is one of an axial-flow impeller structure, a thrombolysis mesh basket structure and a rotary ablation structure.

Further, the interventional instrument further includes a perfusion liquid path. The liquid inlet and the liquid outlet of the perfusion liquid path are provided at the proximal end section of the catheter close to the handle structure. The rotating shaft is configured as a hollow channel or is provided with an axial hole channel. The channel or the hole channel of the rotating shaft enables the perfusion liquid path to be communicated with the injection port of the hydraulic cavity.

Further, the catheter of the interventional instrument is a multi-cavity pipe. The flexible transmission shaft and the perfusion liquid path are provided in different cavities in the catheter.

Further, the interventional instrument further includes an outer cover structure (such as an expandable bracket 3) for supporting and maintaining the cavity for the functional unit. The rotating shaft support structure is configured at the distal end section of the rotating shaft. The distal end section of the outer cover structure is fixedly connected with the rotating shaft support structure, and the proximal end section of the outer cover structure is fixedly connected with the distal end section of the catheter.

Further, the interventional instrument further includes a distal end support structure (such as a pigtail pipe 1). The rotating shaft support structure and the distal end support structure are integrally provided. The distal end section of the outer cover structure is fixedly connected with the proximal end section of the distal end support structure. The proximal end section of the outer cover structure is fixedly connected with the distal end section of the catheter.

Further, the interventional instrument is configured to pump blood in the heart or aspirate thrombus in the blood vessel cavity.

### Application Example 20

After in-depth and extensive research, the applicant also found that for most interventional instruments that need to rotate through the rotating shaft, the over-bending performance of the rotating shaft (that is, the rotating shaft can adapt to the bending radius of different turns when walking through the tortuous vascular structure) and the rotational stability (that is, the rotating shaft can keep the axis from oscillating and swinging under the rotating operation of the load functional element) are a pair of performances which are difficult to be taken into account at the same time. At the same time, the firmness of the connection between the rotating shaft and the functional component is also one of the main safety factors that needs to be taken into account for the interventional instruments. The excellent over-bending performance requires the rotating shaft to have good flexible bending deformation ability, and the excellent rotational stability requires the rotating shaft to have good bending moment deformation resistance (that is, the rigidity of the shaft lever). In the prior art, such as a heart blood pumping device or a thrombus aspiration device, the length of the rotating shaft is often extremely compressed and shortened in order to take both over-bending performance and the rotational stability into account, abandon the double-end/side radial support/limit, and select the single-end/side radial support/limit. This leads to the following technical problems: the connection firmness is obviously reduced due to the reduction of the connection joint surface between the functional element and the rotating shaft, and the anti-interference performance of the whole functional unit is reduced. Therefore, the present disclosure provides a method of strengthening a rotating shaft of an interventional instrument. The core idea of the method is as follows: two materials with different properties are used for embedded configuration with complementary elasticity and rigidity, so that the rotating shaft can be balanced and improved in the aspects of taking both over-bending performance and the rotational stability into account at the same time. At the same time, the connection firmness between the rotating shaft and the functional element can be improved. For example, an elastic polymer material is embedded in the direction that the outer surface of the rotating shaft with a metal body faces the shaft center (the axis) (it can also be understood that a part of the body of the metal rotating shaft is replaced by elastic polymer materials from the outer surface to the axis). The specific method includes the following steps: etching several grooves or through holes from the outer side of the rotating shaft to the axial direction on a rotating shaft with a body having two end faces and an outer side surface made of metal; arranging grooves or through holes in an array in the axial direction and the circumferential direction of the rotating shaft to change the deflection of the metal rotating shaft; filling the grooves or through holes arranged towards these arrays on the outer side of the rotating shaft with elastic polymer materials and adhering the elastic polymer materials to the wall surfaces of the grooves or through holes. It can be understood that the method has been applied and practiced in Application Example 1 to Application Example 9 of the present disclosure. That is, the groove 17 or the through hole 18 of the impeller rotating shaft 7 on the impeller structure 2 is filled with the hub 9. For the metal rotating shaft with changed deflection and embedded with elastic polymer materials, under the rotating operation, the elastic polymer material in the groove or the through hole can elastically pull and elastically compress the adhered metal wall surface, so that the deflection and oscillation of the rotating shaft off the axis can be suppressed, and the rotating shaft can be quickly corrected in terms of deflection to maintain its rotational stability after being interfered by the bending moment.

Further, the elastic polymer material filling each groove or through hole overflows the metal outer side surface of the metal rotating shaft and is piled up to form an elastic polymer coating layer (such as the hub 9) with a continuous outer side surface.

Further, the elastic polymer coating layer is molded into a three-dimensional contour structure (such as the blade 10) with the function of a pump blade.

Further, the metal rotating shaft is configured as a hollow metal pipe rotating shaft. The pipe wall of the hollow metal pipe rotating shaft is etched to form a gap penetrating through the inner and outer wall surfaces of the pipe wall. The gap is filled with elastic polymer materials, which overflows the gap to form an elastic polymer coating layer of a continuous surface (such as a film layer 19 and a hub 9) on the inner and outer wall surfaces of the pipe wall. The elastic polymer coating layer adheres to the wall surface of the metal pipe. It can be understood that: in Application Example 1, the integrated injection molding process is only one of the means to achieve the combination of the metal rotating shaft and the elastic polymer material.

Further, the hollowed-out gaps of the metal pipe rotating shaft are arranged at intervals in the axial direction of the metal pipe rotating shaft and staggered in the circumferential direction. Alternatively, the hollowed-out gaps extend spirally along the central axis of the metal pipe rotating shaft.

Further, in the axial direction of the metal pipe rotating shaft, only the middle section is etched and hollowed out with gaps, while the two end sections have no hollowed-out gaps.

The "rotating shaft", "metal rotating shaft" and "metal pipe rotating shaft" mentioned in this application example all refer to the shafts used to fixedly connect and support a functional element such as an impeller, a blade, a mesh basket, and a rotary ablation blade, and transmit a torque to these functional elements. The shaft can be an independent shaft from the flexible transmission shaft (used to drive the source or handle structure to transmit a torque to the functional unit, which is referred to as 'flexible transmission shaft' in this application example) or a shaft integrated with the flexible transmission shaft.

Based on the method of strengthening a rotating shaft of an interventional instrument provided by the present disclosure, the applicant further provides:
a functional unit of an interventional instrument, where the functional unit includes a functional element for rotation operation and a metal rotating shaft for supporting the functional element and transmitting a torque to the functional element; the functional element includes a body and a base which are integrated with each other; the outer side surface of the metal rotating shaft is provided with grooves or through holes for changing the bending deflection of the metal rotating shaft in the circumferential direction; the grooves or through holes are arranged in an array in the axial direction of the metal rotating shaft or spirally provided in the axial direction of the metal rotating shaft; the base of the functional element is made of elastic polymer materials; a part of the base of the functional element is embedded in the groove or through hole of the metal rotating shaft and adhered to the wall surface of the groove or through hole; the functional element is fixedly connected with the metal rotating shaft through the base; and the body of the functional element is a three-dimensional contour structure with the function of a pump blade.

Further, the metal rotating shaft is a hollow metal pipe rotating shaft.

Further, a part of the functional element base passes through the through hole of the metal pipe rotating shaft, and a continuous layered structure is formed on the inner wall surface of the metal pipe rotating shaft.

Further, the body of the functional element is a blade, and the base of the functional element is a hub.

Further, the metal pipe rotating shaft includes a first rigid section, a flexible section and a second rigid section which are sequentially provided, the through hole is located in the flexible section, and the flexible section is bendable.

### Application Example 21

A blood pumping device includes a functional unit for pumping blood, a flexible transmission shaft, a catheter and a handle structure; the functional unit includes a metal rotating shaft and an impeller; the metal rotating shaft is configured for supporting the impeller and transmitting a torque to the impeller; the catheter is slender, the proximal end of the catheter is connected with the handle structure, and the distal end of the catheter is provided with a rotating shaft support structure which can provide a radial support to the metal rotating shaft; the flexible transmission shaft passes through the conduit, the distal end of the flexible transmission shaft is connected with the metal rotating shaft, the proximal end of the flexible transmission shaft is connected with the handle structure to transmit the torque output by the handle structure to the metal rotating shaft; the functional unit can perform limited axial displacement with respect to the rotating shaft support structure; the impeller includes a hub and a blade; the outer side surface of the metal rotating shaft is provided with grooves or through holes for changing the bending deflection of the metal rotating shaft in the circumferential direction; the grooves or through holes are arranged in an array in the axial direction of the metal rotating shaft or spirally provided in the axial direction of the metal rotating shaft; the hub is made of elastic polymer materials; a part of the hub is embedded in the groove or through hole of the metal rotating shaft and adhered to the wall surface of the groove or through hole; and the impeller is fixedly connected with the metal rotating shaft through the hub.

Further, a part of the hub passes through the through hole of the metal pipe rotating shaft, a continuous layered structure is formed on the inner wall surface of the metal pipe rotating shaft.

Further, the metal pipe rotating shaft includes a first rigid section, a flexible section and a second rigid section which are sequentially provided, the through hole is located in the flexible section, and the flexible section is bendable.

Further, the rotating shaft support structure at the distal end of the catheter further has an axial thrust support function. The first rigid section or the second rigid section of the metal pipe rotating shaft is provided in the rotating shaft support structure having the axial thrust support.

Further, a hydraulic cavity is provided on the moving joint surface between the rotating shaft support structure and the first rigid section or the second rigid section, so that the metal rotating shaft can be supported by liquid in the radial direction and/or the axial direction to be separated from the moving joint surface of the rotating shaft support structure. The hydraulic cavity is provided with a liquid injection port and an outflow port.

Further, the blood pumping device further includes a perfusion liquid path. The liquid inlet and the liquid outlet of the perfusion liquid path are provided at the proximal end section of the catheter adjacent to the handle structure. The metal rotating shaft is configured as a hollow channel or is provided with an axial hole channel. The channel or the hole channel of the metal rotating shaft enables the perfusion liquid path to be communicated with the injection port of the hydraulic cavity.

Further, the catheter of the blood pumping device is a multi-cavity pipe. The flexible transmission shaft and the perfusion liquid path are provided in different cavities in the catheter.

Further, the blood pumping device further includes an outer cover structure (such as an expandable bracket 3) for supporting and maintaining the cavity for the functional unit. The rotating shaft support structure is configured at the distal end section of the metal rotating shaft. The distal end section of the outer cover structure is fixedly connected with the rotating shaft support structure, and the proximal end section of the outer cover structure is fixedly connected with the distal end section of the catheter.

Further, the blood pumping device further includes a distal end support structure (such as a pigtail pipe 1). The rotating shaft support structure and the distal end support structure are integrally provided. The distal end section of the outer cover structure is fixedly connected with the proximal end section of the distal end support structure. The proximal end section of the outer cover structure is fixedly connected with the distal end section of the catheter.

In the present disclosure, the proximal end refers to the end near the doctor, and the distal end refers to the end far away from the doctor.

In this specification, specific examples are used to explain the principle and implementation of the present disclosure.

## Claims

1. A transmission sealing structure for interventional instruments in which a mechanical transmission source and functional elements are configured to be located in use of the interventional instruments, respectively, outside and inside a human body, and the functional elements are configured to communicate with blood vessels to be in contact with blood, the transmission sealing structure comprising an external magnetic component (41), an internal magnetic component (42), a driven shaft (43) and a sealing cover (44), wherein the internal magnetic component (42) is provided on the driven shaft (43), the external magnetic component (41) is provided at an outer side of the internal magnetic component (42), the sealing cover (44) includes an opening end (47) that is hermetically connected with a perfusion chamber (38) by adhesion and an outer wall that is hermetically connected with the perfusion chamber (38) through interference fit so as to isolate the internal magnetic component (42) from the external magnetic component (41), and the external magnetic component (41) is configured to rotate to drive the internal magnetic component (42) and the driven shaft (43) to rotate with respect to the sealing cover (44);
**characterized in that** the internal magnetic component (42) is sleeved on the driven shaft (43) and is fixedly connected with the driven shaft (43), the sealing cover (44) is provided at the outer side of the internal magnetic component (42), the external magnetic component (41) is provided at an outer side of the sealing cover (44), and both the driven shaft (43) and the internal magnetic component (42) are movable in an axial direction in the sealing cover (44);
wherein the external magnetic component (41) surrounds coaxially the sealing cover (44) and the sealing cover (44) surrounds coaxially the internal magnetic component (42), and
one end of the driven shaft (43) protrudes from the opening end (47) of the sealing cover (44), another end of the driven shaft is adjacent to a sealing end of the sealing cover, a gap is formed between the driven shaft (43) and the perfusion chamber (38), a gap is formed between the driven shaft (43) and the sealing cover (44), and between the internal magnetic component (42) and the sealing cover (44); wherein
fluid flows to a space formed by the driven shaft (43), the internal magnetic component (42) and the sealing cover (44) from the gap between the driven shaft (43) and the perfusion chamber (38), and then flows out from the gap between the driven shaft (43) and the perfusion chamber (38).

2. The transmission sealing structure according to claim 1, wherein the driven shaft (43) is a solid shaft, and said one end of the driven shaft (43) is connected with a transmission shaft (12).

3. The transmission seal structure according to claim 1, wherein the driven shaft (43) is a hollow shaft, a transmission shaft (12) extends into the driven shaft (43), and the transmission shaft (12) is a hollow shaft or a solid shaft.

4. The transmission sealing structure according to claim 1, wherein the driven shaft (43) is a plain shaft.

5. The transmission sealing structure according to claim 1, wherein the driven shaft (43) comprises a first shaft part (50) and a second shaft part (51), the first shaft part (50) is located inside the sealing cover (44), the second shaft part (51) is located outside the sealing cover (44), a spiral structure (52) is provided at an outer side of the second shaft part (51), and a spiral direction of the spiral structure (52) is opposite to a rotation direction of the driven shaft (43).

6. The transmission sealing structure according to claim 1, wherein each of the internal magnetic component (42) and the external magnetic component (41) comprises magnetic rings, the magnetic rings in the internal magnetic component (42) are provided in an axial direction of the driven shaft (43) or the magnetic rings in the internal magnetic component (42) are sequentially nested in a radial direction of the driven shaft (43), the magnetic rings in the external magnetic component (41) are provided in the axial direction of the driven shaft (43) or the magnetic rings in the external magnetic component (41) are sequentially nested in the radial direction of the driven shaft (43).

7. The transmission sealing structure according to claim 1, wherein both the internal magnetic component (42) and the external magnetic component (41) comprise several magnetic strips uniformly provided in a circumferential direction of the driven shaft (43).

8. The transmission sealing structure according to claim 1, wherein a wear-resistant insert (53) is provided between the driven shaft (43) and the perfusion chamber (38), the wear-resistant insert (53) is fixedly connected with the perfusion chamber (38), the wear-resistant insert (53) is rotatable with respect to the driven shaft (43), and the wear-resistant insert (53) is capable of axially limiting the driven shaft (43).

## Patentansprüche

1. Getriebedichtungsstruktur für interventionelle Instrumente, bei der eine mechanische Antriebsquelle und Funktionselemente so ausgebildet sind, dass sie sich bei der Verwendung der interventionellen Instrumente jeweils außerhalb bzw. innerhalb eines menschlichen Körpers befinden, und die Funktionselemente so ausgebildet sind, dass sie mit Blutgefäßen in Kontakt mit Blut stehen, wobei die Getriebedichtungsstruktur eine äußere Magnetkomponente (41), eine innere Magnetkomponente (42), eine angetriebene Welle (43) und eine Dichtungsabdeckung (44) umfasst, wobei die innere Magnetkomponente (42) an der angetriebenen Welle (43) vorgesehen ist, die äußere Magnetkomponente (41) an einer Außenseite der inneren Magnetkomponente (42) vorgesehen ist, die Dichtungsabdeckung (44) ein Öffnungsende (47) aufweist, das durch Adhäsion hermetisch mit der Perfusionskammer (38) verbunden ist, sowie eine Außenwand aufweist, die durch Presspassung hermetisch mit der Perfusionskammer (38) verbunden ist, um die innere Magnetkomponente (42) von der äußeren Magnetkomponente (41) zu isolieren, und die äußere Magnetkomponente (41) so ausgebildet ist, dass sie sich dreht, um die innere Magnetkomponente (42) und die angetriebene Welle (43) relativ zur Dichtungsabdeckung (44) in Drehung zu versetzen;
**dadurch gekennzeichnet, dass** die innere Magnetkomponente (42) auf die angetriebene Welle (43) aufgeschoben und fest mit der angetriebenen Welle (43) verbunden ist, die Dichtungsabdeckung (44) an der Außenseite der inneren Magnetkomponente (42) vorgesehen ist, die äußere Magnetkomponente (41) an einer Außenseite der Dichtungsabdeckung (44) vorgesehen ist und sowohl die angetriebene Welle (43) als auch die innere Magnetkomponente (42) in der Dichtungsabdeckung (44) in axialer Richtung beweglich sind;
wobei die äußere Magnetkomponente (41) die Dichtungsabdeckung (44) koaxial umgibt und die Dichtungsabdeckung (44) die innere Magnetkomponente (42) koaxial umgibt, und ein Ende der angetriebenen Welle (43) aus dem Öffnungsende (47) der Dichtungsabdeckung (44) herausragt, ein anderes Ende der angetriebenen Welle an ein Dichtungsende der Dichtungsabdeckung angrenzt, ein Spalt zwischen der angetriebenen Welle (43) und der Perfusionskammer (38) gebildet ist, ein Spalt zwischen der angetriebenen Welle (43) und der Dichtungsabdeckung (44) sowie zwischen der inneren Magnetkomponente (42) und der Dichtungsabdeckung (44) gebildet ist; wobei Flüssigkeit aus dem Spalt zwischen der angetriebenen Welle (43) und der Perfusionskammer (38) in einen Raum strömt, der von der angetriebenen Welle (43), der inneren Magnetkomponente (42) und der Dichtungsabdeckung (44) gebildet wird, und anschließend aus dem Spalt zwischen der angetriebenen Welle (43) und der Perfusionskammer (38) austritt.

2. Getriebedichtungsstruktur nach Anspruch 1, wobei die angetriebene Welle (43) eine Vollwelle ist und das eine Ende der angetriebenen Welle (43) mit einer Getriebewelle (12) verbunden ist.

3. Getriebedichtungsstruktur nach Anspruch 1, wobei die angetriebene Welle (43) eine Vollwelle ist, sich eine Getriebewelle (12) in die angetriebene Welle (43) erstreckt und die Getriebewelle (12) eine Hohlwelle oder eine Vollwelle ist.

4. Getriebedichtungsstruktur nach Anspruch 1, wobei die angetriebene Welle (43) eine glatte Welle ist.

5. Getriebedichtungsstruktur nach Anspruch 1, wobei die angetriebene Welle (43) einen ersten Wellenabschnitt (50) und einen zweiten Wellenabschnitt (51) umfasst, der erste Wellenabschnitt (50) sich innerhalb der Dichtungsabdeckung (44) befindet, der zweite Wellenabschnitt (51) außerhalb der Dichtungsabdeckung (44) angeordnet ist, an einer Außenseite des zweiten Wellenabschnitts (51) eine spiralförmige Struktur (52) vorgesehen ist und die Windungsrichtung der spiralförmigen Struktur (52) der Drehrichtung der angetriebenen Welle (43) entgegengesetzt ist.

6. Getriebedichtungsstruktur nach Anspruch 1, wobei sowohl die innere Magnetkomponente (42) als auch die äußere Magnetkomponente (41) Magnetringe umfassen, die Magnetringe in der inneren Magnetkomponente (42) in axialer Richtung der angetriebenen Welle (43) angeordnet sind oder die Magnetringe in der inneren Magnetkomponente (42) in radialer Richtung der angetriebenen Welle (43) nacheinander ineinander verschachtelt sind, die Magnetringe in der äußeren Magnetkomponente (41) in axialer Richtung der angetriebenen Welle (43) angeordnet sind oder die Magnetringe in der äußeren Magnetkomponente (41) in radialer Richtung der angetriebenen Welle (43) nacheinander ineinander verschachtelt sind.

7. Getriebedichtungsstruktur nach Anspruch 1, wobei sowohl die innere Magnetkomponente (42) als auch die äußere Magnetkomponente (41) mehrere Magnetstreifen umfassen, die gleichmäßig in Umfangsrichtung der angetriebenen Welle (43) angeordnet sind.

8. Getriebeabdichtungsstruktur nach Anspruch 1, wobei zwischen der angetriebenen Welle (43) und der Perfusionskammer (38) ein verschleißfester Einsatz (53) vorgesehen ist, der verschleißfeste Einsatz (53) fest mit der Perfusionskammer (38) verbunden ist, der verschleißfeste Einsatz (53) gegenüber der angetriebenen Welle (43) drehbar ist und der verschleißfeste Einsatz (53) die angetriebene Welle (43) axial begrenzen kann.

## Revendications

1. Structure d'étanchéité de transmission pour des instruments interventionnels dans lesquels une source de transmission mécanique et des éléments fonctionnels sont configurés pour être situés, lors de l'utilisation des instruments interventionnels, respectivement à l'extérieur et à l'intérieur d'un corps humain, et les éléments fonctionnels sont configurés pour communiquer avec des vaisseaux sanguins afin d'être en contact avec le sang, la structure d'étanchéité de transmission comprenant un composant magnétique externe (41), un composant magnétique interne (42), un arbre entraîné (43) et un capot d'étanchéité (44), dans laquelle le composant magnétique interne (42) est disposé sur l'arbre entraîné (43), le composant magnétique externe (41) est disposé à l'extérieur du composant magnétique interne (42), le capot d'étanchéité (44) comprend une extrémité d'ouverture (47) qui est reliée de manière hermétique à une chambre de perfusion (38) par collage et une paroi extérieure qui est reliée de manière hermétique à la chambre de perfusion (38) par ajustement serré de manière à isoler le composant magnétique interne (42) du composant magnétique externe (41), et le composant magnétique externe (41) est configuré pour tourner afin d'entraîner en rotation le composant magnétique interne (42) et l'arbre entraîné (43) par rapport au capot d'étanchéité (44) ;
**caractérisée en ce que** le composant magnétique interne (42) est emmanché sur l'arbre entraîné (43) et est relié fixement à l'arbre entraîné (43), le capot d'étanchéité (44) est disposé à l'extérieur du composant magnétique interne (42), le composant magnétique externe (41) est disposé à l'extérieur du capot d'étanchéité (44), et l'arbre entraîné (43) et le composant magnétique interne (42) sont tous deux mobiles axialement dans le capot d'étanchéité (44) ;
dans laquelle le composant magnétique externe (41) entoure coaxialement le capot d'étanchéité (44) et le capot d'étanchéité (44) entoure coaxialement le composant magnétique interne (42), et
une extrémité de l'arbre entraîné (43) fait saillie depuis l'extrémité d'ouverture (47) du capot d'étanchéité (44), une autre extrémité de l'arbre entraîné est adjacente à une extrémité d'étanchéité du capot d'étanchéité,
un interstice est ménagé entre l'arbre entraîné (43) et la chambre de perfusion (38), et un interstice est ménagé entre l'arbre entraîné (43) et le capot d'étanchéité (44), ainsi qu'entre le composant magnétique interne (42) et le capot d'étanchéité (44) ; dans laquelle un fluide s'écoule vers un espace formé par l'arbre entraîné (43), le composant magnétique interne (42) et le capot d'étanchéité (44), depuis l'interstice entre l'arbre entraîné (43) et la chambre de perfusion (38), puis s'écoule hors de l'interstice entre l'arbre entraîné (43) et la chambre de perfusion (38).

2. Structure d'étanchéité de transmission selon la revendication 1, dans laquelle l'arbre entraîné (43) est un arbre plein, et ladite extrémité de l'arbre entraîné (43) est reliée à un arbre de transmission (12).

3. Structure d'étanchéité de transmission selon la revendication 1, dans laquelle l'arbre entraîné (43) est un arbre creux, un arbre de transmission (12) s'étend dans l'arbre entraîné (43), et l'arbre de transmission (12) est un arbre creux ou un arbre plein.

4. Structure d'étanchéité de transmission selon la revendication 1, dans laquelle l'arbre entraîné (43) est un arbre lisse.

5. Structure d'étanchéité de transmission selon la revendication 1, dans laquelle l'arbre entraîné (43) comprend une première partie d'arbre (50) et une deuxième partie d'arbre (51), la première partie d'arbre (50) est située à l'intérieur du capot d'étanchéité (44), la deuxième partie d'arbre (51) est située à l'extérieur du capot d'étanchéité (44), une structure hélicoïdale (52) est disposée à l'extérieur de la deuxième partie d'arbre (51), et un sens d'enroulement de la structure hélicoïdale (52) est opposé à un sens de rotation de l'arbre entraîné (43).

6. Structure d'étanchéité de transmission selon la revendication 1, dans laquelle le composant magnétique interne (42) et le composant magnétique externe (41) comprennent chacun des anneaux magnétiques, les anneaux magnétiques dans le composant magnétique interne (42) sont disposés dans une direction axiale de l'arbre entraîné (43) ou les anneaux magnétiques dans le composant magnétique interne (42) sont emboîtés successivement dans une direction radiale de l'arbre entraîné (43), les anneaux magnétiques dans le composant magnétique externe (41) sont disposés dans la direction axiale de l'arbre entraîné (43) ou les anneaux magnétiques dans le composant magnétique externe (41) sont emboîtés successivement dans la direction radiale de l'arbre entraîné (43).

7. Structure d'étanchéité de transmission selon la revendication 1, dans laquelle le composant magnétique interne (42) et le composant magnétique externe (41) comprennent tous deux plusieurs bandes magnétiques disposées uniformément dans une direction circonférentielle de l'arbre entraîné (43).

8. Structure d'étanchéité de transmission selon la revendication 1, dans laquelle un insert résistant à l'usure (53) est disposé entre l'arbre entraîné (43) et la chambre de perfusion (38), l'insert résistant à l'usure (53) est relié fixement à la chambre de perfusion (38), l'insert résistant à l'usure (53) peut tourner par rapport à l'arbre entraîné (43), et l'insert résistant à l'usure (53) est apte à limiter axialement l'arbre entraîné (43).
